# EUROPEAN PATENT SPECIFICATION

(11) **EP 0 303 283 B1**
(45) Date of publication and mention of the grant of the patent: **03.02.1993**
(21) Application number: 88113138.7
(22) Date of filing: 12.08.1988
(51) Int. Cl.: C07C 243/24, C07C 241/00, C07C 311/49, C07C 251/74, C07C 251/86, C07C 249/16, C07C 281/06, C08K 5/16, C08K 5/24

(54) **2-Hydroxybenzophenone hydrazides and derivatives thereof**
2-Hydroxybenzophenon-Hydrazide und Derivate davon
Hydrazides de 2-hydroxybenzophénone et dérivés

(30) Priority: 12.08.1987 US 84599
(43) Date of publication of application: 15.02.1989
(73) Proprietor: ATOCHEM NORTH AMERICA, INC., Philadelphia, Pennsylvania 19102 (US)
(72) Inventor: Myers, Terry Ned, Williamsville New York 14221 (US)
(74) Representative: Abitz, Walter, Dr.-Ing.

(56) References cited:
- WO-A-86/03760
- GB-A- 1 329 847
- GB-A- 2 136 796

## Description

The present invention relates to certain compounds which incorporate both o-hydroxybenzophenone (an ultraviolet light stabilizer) and hydrazide (a heat stabilizer) functional groups.

In addition to activity as a stabilizer on a molar basis (i.e., UV absorber, antioxidant, etc.), a successful stabilizer additive must have both excellent compatibility with and/or solubility in numerous polymer substrates along with superior resistance to loss from the stabilized composition during processing and end-use application. Many stabilizer additives exhibit limited compatibility in certain substrates, and excessive tendency to exude, sublime and/or volatilize during weathering or processing of stabilized compositions, particularly when use conditions require exposure to elevated temperatures. Because of this problem, several attempts have been made to increase the compatibility and reduce the volatility of such stabilizer additives by modifying their structure. While improvements have been noted over the years, experience has shown that state-of-the-art stabilizers do not exhibit the desired combination of properties in all resins and that new polymeric compositions continue to invoke additional structural modifications on any potential heat and/or light stabilizer intended for use. Two examples of this would be in "high solids" coatings which require greater solubility of the stabilizer due to the use of less solvent, and in engineering thermoplastics where processing temperatures (in excess of 250°C) require the use of stabilizers with high thermal stability and very low volatility. Obviously, no one stabilizer to date provides the properties necessary for universal application and there is a constant commercial need for new stabilizers offering a range of property advantages.

Systems which incorporate UV absorbers and other functional groups are known. Multifunctional stabilizers have been prepared by reacting one type of stabilizer with another to obtain a higher molecular weight compound having dual functionality or by reacting two or more stabilizers with a multifunctional coupling agent (e.g., cyanuric chloride) in a stepwise fashion. U.S. Patent 4,481,315 discloses molecular combinations of hydroxybenzophenones and polyalkylpiperidines. Examples of some other stabilization systems contained in one molecule can be found in U.S. Patents 3,536,661 and 4,198,334. Japanese Patent 73/43568 (CA81: 122589s) discloses the use of a 2-(2-hydroxyphenyl)-2H-benzotriazole UV absorber and a hydrazide to give enhanced resistance to photodegradation of polyurethane copolymer fibers. None of this prior art discloses the present invention.

### SUMMARY OF THE INVENTION

This invention is directed to a compound of the formula: 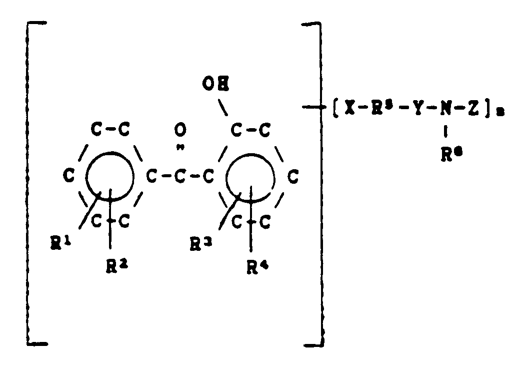
wherein:
R¹, R², R³ and R⁴ are independently selected from hydrogen, alkyl of 1 to 10 carbons, acyl of 1 to 10 carbons, alkoxy of 1 to 18 carbons, aryl of 6 to 10 carbons, aralkyl of 7 to 16 carbons, alkoxycarbonyl of 2 to 19 carbons, aryloxy of 6 to 10 carbons, alkylmercapto of 1 to 10 carbons, arylmercapto of 6 to 10 carbons, alkylamino of 1 to 5 carbons, dialkylamino of 2 to 10 carbons total, arylamino of 6 to 10 carbons, aryl alkyl amino of 7 to 20 carbons total, cycloalkyl of 3 to 12 carbons, aroyl of 7 to 11 carbons, carboxy, hydroxy, chloro, bromo, cyano, carbamyl, sulfamyl, alkylcarbamyl of 1 to 10 carbons, alkyl sulfamyl of 1 to 10 carbons, alkoxycarbonyloxy of 2 to 11 carbons, and acyloxy of 1 to 10 carbons.

Optional substituents for R¹, R², R³ and R⁴ are selected from chloro, bromo, cyano, nitro, mercapto, alkylmercapto of 1 to 4 carbons, alkoxy of 1 to 4 carbons, hydroxy, carboxy, carbonyl, alkoxy carbonyl of 2 to 12 total carbons, and carboxylate. Preferably, R¹, R², R³ and R⁴ are selected from hydrogen, alkyl of 1 to 4 carbons, alkoxy of 1 to 12 carbons, aryl of 6 carbons, aralkyl of 7 to 9 carbons, carboxy, hydroxy or chloro with optional substituents for the alkyl, alkoxy, aryl and aralkyl being selected from chloro, bromo, alkylmercapto of 1 to 4 carbons, hydroxy, carboxy, and alkoxycarbonyl of 2 to 6 total carbons. Most preferably, R¹, R², R³ and R⁴ are selected from hydrogen, alkoxy of 1 to 4 carbons, hydroxy, and chloro with optional substituents for the alkoxy group being selected from chloro, bromo, alkyl mercapto of 1 to 2 carbons, and hydroxy.

n is 1 or 2. Preferably, n is 1.

X is -O-, -N(R⁶)-, -S-, -O-C(=O)- or a bond between the aromatic ring and R⁵. Preferably, X is selected from -O-, -O-C(=O)-, and a direct bond between the ring and R⁵. Most preferably, X is -O- or a direct bond between the ring and R⁵.

R⁵ is selected from alkylene of 1 to 10 carbons, alkenylene of 2 to 10 carbons, 1,4 or 1,3 or 1,2-phenylene, or cycloalkanediyl of 5 to 12 carbons. Optional substituents for these diradicals are selected from cyano, chloro, bromo, alkyl of 1 to 4 carbons, acyl of 1 to 4 carbons, alkoxy of 1 to 4 carbons, carboxy, alkoxycarbonyl of 2 to 6 carbons total, alkoxyalkyl of 2 to 6 carbons, or alkoxyalkenoxy of 3 to 6 carbons. Preferably, R⁵ is selected from alkylene of 1 to 8 carbons, alkenylene of 2 to 6 carbons, or 1,2 or 1,3 or 1,4 phenylene with optional substituents for these groups selected from chloro, bromo, alkyl of 1 to 4 carbons, carboxy, alkoxy of 1 to 4 carbons, and alkoxycarbonyl of 2 to 6 carbons. Most preferably, R⁵ is alkylene of 1 to 6 carbons, with optional substituents chosen from chloro, bromo, alkyoxy of 1 to 4 carbons, hydroxy, and carboxy.

Y is selected from -C(=O)-, -S(=O)₂-, and -OC(=O)-.

R⁶ is selected from hydrogen, alkyl of 1 to 10 carbons, aryl of 6 to 10 carbons, acyl of 1 to 10 carbons, or aroyl of 7 to 11 carbons. Preferably, R⁶ is selected from hydrogen, alkyl of 1 to 4 carbons, acyl of 1 to 6 carbons, and aroyl of 7 to 9 carbons. Most preferably, R⁶ is selected from hydrogen, alkyl of 1 to 4 carbons, or acyl of 1 to 4 carbons.

Z is selected from: -N(Q-R⁷)(R⁸), -N=C(R⁹)(R¹⁰),

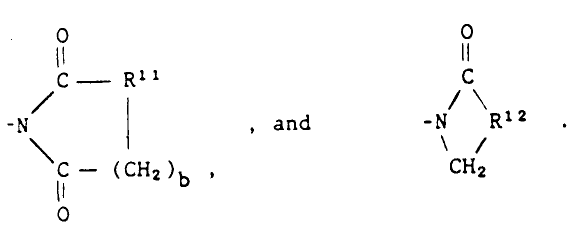

Preferably, Z is selected from: -N(Q-R⁷)(R⁸), -N=C(R⁹)(R¹⁰),

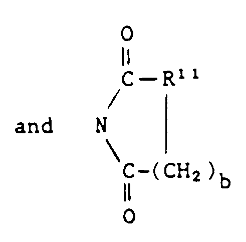

Q is selected from -C(=O)-, -S(=O)₂-, -C(=O)-O-, or -C(=O)-N(R⁸)-, and a direct bond between the nitrogen and R⁷. Preferably, Q is selected from -C(=O)-, -C(=O)-O-, -C(=O)-N(R⁸)- and a direct bond between the nitrogen and R⁷. Most preferably, Q is -C(=O)-, -C(=O)-O-, and a direct bond between the nitrogen and R⁷.

R⁷ and R⁸ are independently selected from hydrogen, alkyl of 1 to 20 carbons, substituted or unsubstituted alkenyl of 2 to 6 carbons, aralkyl of 7 to 12 carbons, cycloalkyl of 5 to 12 carbons, substituted or unsubstituted aryl of 6 to 14 carbons, and polyoxyalkylene of formula 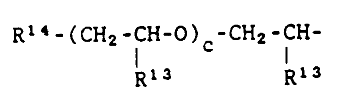
wherein R¹⁴ is selected from alkoxy of 1-20 carbons, aryloxy of 6-10 carbons and alkoxy-alkoxy of 3-20 carbons, alkyl mercapto of 1-6 carbons and alkenyloxy of 3-7 carbons, R¹³ is selected from methyl and hydrogen, and c is an integer 2 to 50, polyalkyl of the structure CH₃-(CH₂)_{d}- in which d is an integer 25 to 50, and substituted triazines of structure 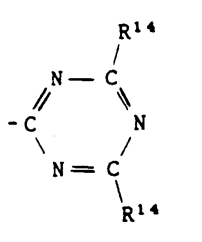
Preferably, R⁷ is selected from hydrogen, alkyl of 1 to 8 carbons, polyoxyalkylene, substituted or unsubstituted alkenyl of 2 to 6 carbons, or substituted or unsubstituted aryl of 6 to 14 carbons, and R⁸ is hydrogen, alkyl of 1 to 8 carbons, or aryl of 6 to 10 carbons. Most preferably, R⁷ is selected from hydrogen, alkyl of 1 to 4 carbons, polyoxyethylene, or substituted or unsubstituted aryl of 6 to 14 carbons, and R⁸ is hydrogen, alkyl of 1 to 4 carbons, or aryl of 6 carbons.

b is 0 or 1. Preferably, b is O.

R⁹ and R¹⁰ are independently selected from hydrogen, alkyl of 1 to 10 carbons, and aryl of 6 to 10 carbons. R⁹ and R¹⁰ may be joined to form a ring of 5 to 12 carbons which may optionally contain unsaturation and heteroatoms chosen from nitrogen, oxygen and sulfur. Preferably, R⁹ and R¹⁰ are chosen from hydrogen, alkyl of 1 to 8 carbons, and a connected species forming a ring of 5 to 7 carbons optionally containing unsaturation and heteroatoms chosen from nitrogen, oxygen and sulfur. Most preferably, R⁹ and R¹⁰ are chosen from hydrogen, and a connected species forming a ring optionally containing unsaturation and heteroatoms such as nitrogen, oxygen and sulfur.

R¹¹ and R¹² are independently selected from alkylene of 2 to 5 carbons, alkenylene of 2 to 3 carbons, aryl diradical of 6 to 12 carbons, and aralkyl diradical of 7 to 15 carbons wherein the alkylene and alkylene diradicals may optionally contain heteroatoms chosen from nitrogen, oxygen and sulfur. Preferably, R¹¹ and R¹² are selected from alkylene of 2 to 5 carbons, alkenylene of 2 to 3 carbons, and aryl diradical of 6 to 12 carbons wherein the alkylene and alkenylene diradicals may optionally contain heteroatoms chosen from nitrogen, oxygen and sulfur. Most preferably, R¹¹ and R¹² are selected from alkylene of 2 to 3 carbons, alkenylene of 2 carbons, and aryl diradical of 6 to 12 carbons wherein the alkylene and alkenylene diradicals may optionally contain heteroatoms chosen from nitrogen, oxygen and sulfur.

Substituents for R⁷, R⁸, R⁹, R¹⁰ and R¹¹ are selected from one or more hydroxy, chloro, bromo, cyano, nitro, carboxy, alkoxy of 1 to 4 carbons, alkoxycarbonyl of 2 to 6 carbons, and alkyl of 1 to 4 carbons.

### DETAILED DESCRIPTION OF THE INVENTION

This invention comprises molecular combinations of 2-hydroxybenzophenone UV stabilizers and hydrazide antioxidants/heat stabilizers/metal deactivators. Such combinations are unique compounds incorporating multiple stabilizer functional groups. By manipulation of the hydrazide group, additional useful functional groups can be attached without compromising the beneficial effect of the hydrazide. This aspect broadens the scope of the invention significantly. All the compounds described in this disclosure are additives for polymers providing light, thermal and oxidative stabilization.

Illustrative examples of compounds of the present invention are as follows:
1. 2-(4-benzoyl-3-hydroxyphenoxy)propionyl hydrazide,
2. 4-(4-methylbenzoyl)-3-hydroxyphenoxyacetyl hydrazide,
3. 4-(2,4-dimethoxybenzoyl)-3-hydroxyphenoxyacetyl hydrazide,
4. 4-(4-benzoyl-3-hydroxyphenoxy)butanoyl hydrazide,
5. N-[3-(4-benzoyl-3-hydroxyphenoxy)propionyl]-N-methylhydrazine,
6. N-4-(4-ethoxybenzoyl)-3-hydroxyphenoxyacetyl]-N'-(2-hydroxypropyl)hydrazine,
7. N-(4-benzoyl-3-hydroxyphenoxyacetyl)-N'(2-carboxybenzoyl)hydrazine,
8. N-[4-(2,4-dichlorobenzoyl )-3-hydroxyphenoxyacetyl]-N'-(6-carboxy-4-thiahexanoyl)hydrazide,
9. N-(5-benzoyl-4-hydroxy-2-methoxybenzenesulfonyl)-N'-phenylhydrazine,
10. N-4-[4-dimethylamino)benzoyl]-3-hydroxyphenoxyacetyl-N'-(2-phenoxyethoxycarbonyl)hydrazine, and
11. N-[(4-benzoyl-3-hydroxyphenoxy)acetamido]tetrabromophthalimide.

The 2-hydroxybenzophenone derivatives of this invention can be used to protect polymeric materials from photooxidative degradation. The stabilizers may be incorporated into the polymer at any effective concentration, generally 0.01 to 5% by weight based on the weight of polymer to be stabilized, preferably 0.05 to 3% by weight. In some cases 0.5 to 1% by weight of the final polymer composition is sufficient.

In addition, the hydrazide funtionalized 2-hydroxybenzophenone stabilizers are reactive additives that can be attached to co-reactive polymers to form polymer bound additives containing photooxidative and thermal oxidative stabilizing groups. Once reacted with the co-reactive polymers, the stabilizer groups become chemically bound to the polymers and will not be lost via volatilization, migration or extraction.

Example of polymers and copolymers which may be stabilized by the compound of this invention include:
1. Polyolefins such as high, low and linear low density polyethylenes, which may be optionally crosslinked, polypropylene, polyisobutylene, poly(methylbutene-1), polyacetylene and in general polyolefins derived from monomers having from two to about ten carbon atoms and mixtures thereof.
2. Polyolefins derived from diolefins such as polybutadiene and polyisoprene.
3. Copolymers of mono or diolefins such as ethylene-propylene, propylene-butene-1 copolymer.
4. Terpolymers of ethylene and propylene with dienes (EPDM) such as butadiene, hexadiene, dicyclopentadiene and ethylidene norbornene.
5. Copolymers of alpha-olefins with acrylic acid or methacrylic acids or their derivatives such as ethylene-acrylic acid, ethylene-methacrylic acid and ethylene-ethyl acrylate copolymers.
6. Styrenic polymers such as polystyrene (PS) and poly(p-methylstyrene).
7. Styrenic coplymers and terpolymers such as styrene-butadiene (SBR), styrene-allyl alcohol and styrene-acrylonitrile (SAN), styrene-acrylonitrile-methacrylate terpolymer, styrene-butadiene-styrene block copolymers (SBS), rubber modified styrenics such as styrene-acrylonitrile copolymers modified with acrylic ester polymer (ASA), graft copolymers of styrene on rubbers such as polybutadiene (HIPS), polyiosprene or styrene-butadiene-styrene block copolymers, graft copolymers of styrene-acrylonitrile on rubbers such as butadiene (ABS), polyisoprene or styrene-butadiene-styrene block copolymers, graft copolymers of styrene-methyl methacrylate on rubbers such as polybutadiene (MBS), butadiene-styrene radial block copolymers (e.g., KRO 3 of Phillips Petroleum Co.), selectively hydrogenated butadiene-styrene block copolymers (e.g., Kraton G from Shall Chemical Co.) and mixtures thereof.
8. Polymers and copolymers derived from halogen-containing vinyl monomers such as poly(vinyl chloride), poly(vinyl fluoride), Poly(vinylidene fluoride),poly(tetrafluoroethylene) (PTFE), vinyl chloride-vinyl acetate copolymers, vinylidene chloride-vinyl acetate copolymers and ethylenetetrafluoroethylene copolymers.
9. Halogenated rubbers such as chlorinated and/or brominated butyl rubbers such as chlorinated and fluoroelastomers.
10. Polymers and copolymers derived from alpha, beta-unsaturated acids, anhydrides, ester, amides and nitriles or combinations thereof such as polymers or copolymers of acrylic and methacrylic acids, alkyl and/or glycidyl acrylates and mathacrylates, acrylamide and methacrylamide, acrylonitrile, maleic anhydride, maleimide, the various anhydride containing polymers and copolymers described in this disclosure, copolymers of the above polymers and various blends and mixtures thereof as well as rubber modified versions of the above polymers and copolymers.
11. Polymers and copolymers derived from unsaturated alcohols or their acylated derivatives such as poly(vinyl alcohol), poly(vinyl acetate), poly(vinyl stearate, poly(vinyl benzoate), poly(vinyl maleate, poly(vinyl butyral), poly(allyl phthalate), poly(allyl diethylene glycol carbonate) (ADC), ethylene-vinyl acetate copolymer and ethylene-vinyl alcohol copolymers.
12. Polymers and copolymers derived from unsaturated amines such as poly(allyl melamine).
13. Polymers and copolymers derived from epoxides such as polyethylene oxide, polypropylene oxide and from bis-glycidyl ethers.
14. Poly(phenylene oxides, poly(phenylene ethers) and modifications thereof containing grafted polystyrene or rubbers as well as their various blends with polystyrene, rubber modified polystyrenes or nylon.
15. Polycarbonates and especially the aromatic polycarbonates such as those derived from phosgene and bisphenols such as bisphenol-A, tetrabromobisphenol-A and tetramethylbisphenol-A.
16. Polyester derived from dicarboxylic acids and diols and/or hydroxycarboxylic acids or their corresponding lactones such as polyalkylene phthalates (e.g., polyethylene terephthalate (PET), polybutylene terephthalate (PBT), and poly(1,4-dimethylcyclohexane terephthalate) or copolymers thereof) and polylactones such as polycaprolactone.
17. Polyarylates derived from bisphenols (e.g., bisphenol-A and various aromatic acids such as isophthalic and terephthalic acids or mixtures thereof.
18. Aromatic copolyestercarbonates having carbonate as well as ester linkages present in the backbone of the polymers such as those derived from bisphenols, iso- and terephthaloyl chlorides and phosgene.
19. Polyurethanes and polyureas.
20. Polyacetals such as polyoxymethylenes and polyoxymethylenes which contain ethylene oxide as a comonomer.
21. Polysulfones, polyethersulfones and polyimidesulfones.
22. Polyamides and copolyamides which are derived from diamines and dicarboxylic acids and/or from aminocarboxylic acids or the corresponding lactones such as the following nylons: 6, 6/6, 6/10, 11, and 12.
23. Polyimides, polyetherimides, polyamideimides and copolyetheresters.
24. Cross-linked polymers which are derived from aldehydes on the one hand and from phenols, ureas and melamine on the other hand such as phenolformaldehyde, urea-formaldehyde and melamine-formaldehyde resins.
25. Alkyl resins such as glycerol-phthalic acid resins and mixtures thereof with melamine-formaldehyde resins.
26. Unsaturated polyester resins which are derived from copolyesters of saturated and unsaturated dicarboxylic acids with polyhydric alcohols as well as from vinyl comopunds (crosslinking agents) and also halogen-containing, flame resistant modifications thereof.
27. Natural polymers such as cellulose, natural rubber as well as the chemically modified homologous derivatives thereof such as cellulose acetates, cellulose propionate, cellulose butyrate and the cellulose.

In addition, the stabilizers of this invention may be used to stabilize various combinations or blends of the above polymers or copolymers. They are particularly useful in the stabilization of polyolefins, acrylic coatings, styrenics, rubber modified styrenics, poly(phenylene oxides) and their various blends with styrenics, rubber-modified styrenics or nylon.

### Preparative methods

The following list includes the basic procedures useful for the preparation of the compounds of this invention:
1) Reaction of an ester with hydrazine or an alkyl hydrazine in a solvent (usually aqueous alcohol) and at sufficient temperature to convert the ester into the corresponding hydrazide.
2) Reaction of a ketone or aldehyde in the absence of water in a suitable solvent (toluene, xylene, often simply an excess of the ketone or aldehyde) with a hydrazide in the presence of absence of an acid catalyst at a suitable temperature (refluxing solvent) to cause the formation of the hydrazone of the carbonyl compound. Water is often removed from the reaction as it forms by azeotropic distillation.
3) Acylation of a hydrazide by an acylating agent. The acylating agent is typically an acid chloride, chloroformate, ester, anhydride or isocyanate. The presence of a base either in catalytic (for anhydride and isocyanate reaction) or stoichiometric (for acid chloride and chloroformate reaction) amount may be used. The reaction is conducted at a temperature sufficient for reaction to occur.
4) Alkylation of a hydrazide by an alkylating agent. The alkylating agent is typically an alkyl halide or epoxide. The reaction is usually conducted in solvent (possibly excess alkylating agent) and optionally in the presence of a base to scavenge the halogen acid if formed by the reaction. Temperatures for this reaction vary with the activity of the alkylating agent.
5) Cyclization of an amide/acid or amide/ester. The product of reactions 1) and 3) above may result in the formation of a molecule containing both an amide group and an ester or acid group. In this case, the amide may undergo further reaction with the acid or ester to form a cyclic structure called an imide. If the requisite functional groups are present in the necessary proximity, the reaction to form the imide occurs simply upon prolonging the reaction time and increasing the temperature. In some instances the addition of an acid catalyst facilitates the reaction. In some instances it may be necessary to promote the reaction by the addition of another reactant such as acetic anhydride which is known to promote ring closure of amic acids to imides.

As an illustration, a versatile compound is obtained by reaction of ethyl (4-benzoyl-3-hydroxy-phenoxy)acetate with hydrazine in aqueous alcohol for a few hours at ambient temperature followed by dilution of the reaction mixture with additional water and filtration of the solid (4-benzoyl-3-hydroxy-phenoxy)acetyl hydrazide. This hydrazide can be reacted with acylating agents such as acid chlorides, anhydrides, esters, chloroformates and isocyanates to form many multifunctional derivatives. Also, the hydrazide can be reacted with carbonyl compounds such as ketones and aldehydes to yield hydrazones. Many of the possibilities discussed here are illustrated by the following examples.

The following examples are presented to provide illustrations and not limitations of the present invention. Unless otherwise stated herein, the temperatures are in degrees Centigrade and all parts are by weight.

### EXAMPLE I

### Preparation of Carboxylic Acid Hydrazide Containing 2-Hydroxybenzophenones

Ethyl 4-benzoyl-3-hydroxyphenoxyacetate (30.0g, 0.1 mole) and 85% hydrazine hydrate (120 ml aq. solution, 2.0 moles of hydrazine, pure basis) were stirred in 300 ml of 2-propanol at ambient temperature for three hours. The reaction mixture was poured into 1 liter of deionized water containing acetic acid (116.0g, 1.9 moles) and cooled in an ice bath. The solid precipitate was collected on a Buchner funnel, washed with additional dionized water, then slurried with a small amount of tetrahydrofuran before a final filtration. The isolated solid was dried overnight in a vacuum oven. The 4-benzoyl-3-hydroxyphenoxyacetyl hydrazide (Ia) thus obtained weighed 19.7g (0.07 mol, 70% of theory) and had a melting range of 201-203°C. Infrared spectroscopy showed multiple ketone carbonyls around 1635 cm⁻¹, the hydrazide carbonyl at 1675 cm⁻¹, and a broad combined OH and NH absorption of 2800-3400 cm⁻¹. The structure was further confirmed using 1H and 13C NMR spectroscopy and by elemental analysis (theoretical: 62.93% C, 4.93% H, 9.78% N; found: 62.96% C, 4.97% H, 9.71% N).

When ethyl 2-(2,4-dihydroxybenzoyl)benzoate was substituted for ethyl 4-benzoyl-3-hydroxyphenoxyacetate in this preparation, the product was 2-(2,4-dihydroxybenzoyl)-benzoyl hydrazide (Ib, yield 99+%, melting range 286-292°C). Infrared spectral data for this compound showed multiple ketone carbonyls around 1625 cm⁻¹, the hydrazide carbonyl at 1650 cm⁻¹, and a broad combined OH and NH absorption of 2400-3700 cm⁻¹.

### EXAMPLE II

### A. Preparation of 5-benzoyl-4-hydroxy-2-methoxybenzenesulfonyl chloride

Uvinul MS-40, 20.0g, 0.065 mole, was dissolved in 250 ml of warm tetrahydrofuran and cooled to room temperature. A solution of 8.0g (0.10 mole) of pyridine in 25 ml of tetrahydrofuran was added accompanied by an exotherm. The resulting mixture was stirred at room temperature for 30 minutes then poured into 200 ml of pentane and stirred briefly before the sulfonic acid salt (23.5g) was collected by filtration. This salt was added slowly over 15 minutes to 29.0g (0.244 mole) of thionyl chloride at 50°C for 1 hour 45 minutes. The reaction mixture was poured into 60 ml of ice water to precipitate the product. The product was isolated by filtration and transferred to separatory funnel with 200 ml of chloroform. The mixture was extracted with 125 ml of 2.5% HCl, 100ml of 2.5% HCl, and 100 ml of water. The organic was dried with anhydrous magnesium sulfate and stripped of solvent using an aspirator and high vacuum. The product weighed 16.5g and melted at 165-172°C.

### B. Preparation of 5-benzoyl-4-hydoxy-2-methoxybenzenesulfonyl hydrazide

Into a 125 ml 3-neck round bottom flask were placed 2.0g (0.029 mole) of hydrazine monohydrochloride and 25 ml of water. In a separate flask, the sulfonyl chloride, 3.3g (0.01 mole), prepared above, was dissolved in 50 ml of tetrahydrofuran. The acid chloride was added to the hydrazine solution with about 5°C exotherm. The reaction mixture was heated to 55°C and then allowed to stand at room temperature for 60 hours then heated at 55°C for 72 hours. The reaction mixture was transferred to a separatory funnel with 100 ml of tetrahydrofuran and 50 ml of saturated aqueous sodium chloride. The organic phase was separated and the aqueous phase was washed with an additional 100 ml of tetrahydrofuran. The combined organic solutions were dried with anhydrous sodium sulfate and the solvent was stripped using an aspirator and high vacuum. The solid residue was dissolved in 200 ml of methanol and treated with a solution of potassium hydroxide, 0.49g (0.0075 mole), dissolved in 7 ml of methanol. The resulting methanol solution was diluted with 250 ml of tetrahydrofuran and the material which precipitated was isolated by filtration. The solid was slurried with another 250 ml of tetrahydrofuran and filtered again. The solid was then recrystallized from aqueous ethanol. The product melted at >280°C. Infrared spectroscopy showed multiple ketone carbonyls at 1600 and 1625 cm⁻¹, and the sulfonyl group at 1270 cm⁻¹. The structure was further confirmed using proton and carbon-13 NMR spectroscopy.

### EXAMPLE III

### Reaction of Ia with Various Anhydrides

The hydrazide Ia (6.4g, 0.0137 mole), tetrabromophthalic anhydride (3.9g, 0.0137 mole) and 100 ml of xylene were combined in a dry 200 ml flask fitted with a Dean-Stark trap. The reaction mass was heated to reflux, azeotroping the water as it formed. This process was continued for two hours. The reaction mixture was stripped of solvent using aspirator vacuum to give 10.0g of product with a melting point of >280°C. The produce, (IIIa), had an assay for bromine of 41.8%. The infrared spectrum of this compound showed multiple ketone carbonyls around 1620 cm⁻¹, and imide and amic acid carbonyl bands around 1750 cm⁻¹.

The following analogs were also prepared:
From homophthalic anhydride, product IIIb was obtained in 20% yield, mp 80-85°C, infrared bands (carbonyl): 1620,1710 and 1760 cm⁻¹.

From 3-oxaglutaric anhydride, the product IIIc was obtained in 99% yield, mp 185-189°C, infrared carbonyl bands at 1620 and 1700 cm⁻¹.

From 1,8-naththoic anhydride, product IIId was obtained in 84% yield, mp 125-130°C, infrared carbonyl bands at 1620 and 1710 cm⁻¹.

From 1,4,5,6,7,7-hexachloro-5-norbornene- 2,3-dicarboxylic anhydride, the product IIIe was obtained in 88% yield, mp >200°C(d), infrared carbonyl bands at 1625 and 1750 cm⁻¹.

From phthalic anhydride, the product IIIf (99+% yield based on theoretical imide, infrared carbonyl bands 1630, 1700, and 1730 cm⁻¹) was further imidized to a composition melting at 79-83°C, infrared bands of 1625, and 1750 cm⁻¹, by either heating the mixture neat to 320°C or refluxing the mixture in xylene containing acetic anhydride for 2 hours.

### EXAMPLE IV

### Reaction of Ia with Thiodiacetic Anhydride

The hydrazide Ia (3.4g, 0.012 mole), thiodiacetic anhydride (2.0g, 0.014 mole) and 100 ml of xylene were combined in a dry 200 ml flask fitted with a Dean-Stark trap. The reaction mass was heated to reflux, azeotroping the water as it formed. This process was continued for two hours; then the reaction mixture was cooled and the solid product was collected in a Buchner funnel. This solid was mixed with acetic acid (45g) and sodium acetate (2.7g) and the resulting mixture was heated to 70°C for 10 minutes. The reaction was quenched by pouring the reaction mass into water and the solid precipitate was isolated by filtration. The moist product was dissolved in tetrahydrofuran and dried with anhydrous magnesium sulfate. The desiccant and solvent were removed leaving 1.6g of white crystalline solid melting about 60°C. The infrared spectrum of this material showed multiple ketone carbonyls around 1610 cm⁻¹ and an imide carbonyl at 1720 cm⁻¹.

### EXAMPLE V

### Reaction of Ia with Acetic Anhydride

The hydrazide Ia (4.35g, 0.0152 mole) and 250 ml of tetrahydrofuran were combined in a dried 500 ml flask. Acetic anhydride (1.6g, 0.016 mole) dissolved in 50 ml of tetrahydrofuran was added and the reaction mixture was heated to 40°C for 3 hours (the reaction mixture became homogeneous after about 2 hours). The reaction mass was stripped under aspirator vacuum to yield a solid residue. This residue was taken up in sufficient tetrahydrofuran to dissolve the solid at the boiling point and allowed to recrystallize. The crystallized product was collected on a Buchner funnel as 3.2g of slightly yellow crystals. The recrystallization solvent was reduced to about 25 ml and a second crop of crystals was allowed to form (0.9g). A total of 4.1g of product was obtained, melting at 177-179°C.; the yield was 82%. The infrared spectrum showed multiple carbonyl absorptions around 1620 and 1700 cm⁻¹.

### EXAMPLE VI

### Reaction of Ia with Benzoyl Chloride

The hydrazide Ia (3.7g, 0.013 mole), benzoyl chloride (2.0g, 0.014 mole) and 300 ml of tetrahydrofuran were combined in a dried 500 ml flask under under a nitrogen atmosphere. An addition funnel was attached and charged with triethylamine (1.4g, 0.014 mole) and 25 ml of tetrahydrofuran. The amine solution was added dropwise over a five minute period accompanied by an increase in reaction temperature from 20 to 23°C. The reaction mixture was then stirred at ambient temperature for 1 hour. The triethylammonium chloride was filtered off and the liquid filtrate was transferred to a separatory funnel with 50 ml of methyl t-butyl ether. The organic material was washed with 50 ml of 2.5% sodium bicarbonate and then with 50 ml of water. The organic layer was dried using anhydrous sodium sulfate and anhydrous magnesium sulfate. The solvent was stripped using aspirator and high vacuum to give 5.1g of slightly yellow crystals with a melting range of 166-170°C. The product was obtained in 99+% yield. The infrared spectrum of this material showed multiple ketone carbonyl bands around 1630 cm⁻¹ and hydrazide carbonyls merged around 1720 cm⁻¹.

### EXAMPLE VII

### Hydrazones of Ia

The hydrazide Ia (3.7g, 0.013 mole), 2-heptanone (1.6g, 0.014 mole), p-toluenesulfonic acid monohydrate (0.1g) and 100 ml of toluene were combined in a dried 200 ml flask fitted with a Dean-Stark trap. The reaction mixture was heated to reflux and the azeotrope separated for 3 hours (the reaction mixture cleared after 2 hours). The reaction mass was transferred to a separatory funnel with 50 ml of methyl t-butyl ether and 200 ml toluene. The organic mixture was washed with 50 ml of 5% sodium bicarbonate causing the formation of an insoluble interface (starting hydrazide). The aqueous material was drained off and the organic phase and interface was filtered. The organic material was put back in the separatory funnel and washed with 100 ml of saturated sodium chloride. The organic material was dried with anhydrous magnesium sulfate. After removing the desiccant, the solvent was stripped using an aspirator vacuum. The product was 3.9g of white crystals melting at 85-92°C. The infrared spectrum of this material showed multiple ketone carbonyl bands around 1620 cm⁻¹ and the acyl hydrazone carbonyl at 1710 cm⁻¹.

When 3-methylcyclohexanone was used instead of 2-heptanone, the product obtained (VIIIb) melted at about 110°C. The infrared spectrum of this compound showed multiple ketone carbonyls around 1630 cm⁻¹ and the acyl hydrazone carbonyl at 1700 cm⁻¹.

When 2,4-dichlorobenzaldehyde was used instead of 2-heptanone, the product (VIIc), melted at 191-196°C. The infrared spectrum of this compound showed multiple ketone carbonyls around 1630 cm⁻¹ and the acyl hydrazone carbonyl at 1710 cm⁻¹.

### EXAMPLE VIII

### Hydrazone of Ia and 2,2,6,6-Tetramethyl-4-piperidone

The hydrazide Ia (3.4g, 0.012 mole), 2,2,6,6-tetramethyl-piperidone monohydrate (3.0g, 0.017 mole), p-toluene sulfonic acid monohydrate (0.15g) and 100 ml of xylene were combined in a dried 200 ml flask. The reaction mixture was heated to reflux and the azeotrope separated for 10 hours. The reaction mass was cooled and stripped of xylene and the solid residue slurried with 100 ml of pentane and filtered. The product was 2.8g of tan crystals melting at 193-196°C. The infrared spectrum of this compound showed multiple ketone carbonyls around 1630 cm⁻¹ and the acyl hydrazone carbonyl at 1700 cm⁻¹.

### EXAMPLE IX

### Preparation of 4-Benzoyl-3-hydroxy-phenoxy-N'-phenyl acetyl Hydrazide

Ethyl 4-benzoyl-3-hydroxyphenoxyacetate (30.0g, 0.1 mol) and phenyl hydrazine (14 ml, 0.4 mole) were stirred in 100 ml of toluene at 75°C for 3.5 hours, then at 100°C for 13 hours. The reaction mixture was poured into 300 ml of deionized water containing acetic acid (7.6g, 0.1 mole) and stirred. The mixture was transferred to a separatory along with 600 ml of methyl t-butyl ether. At this point, it was necessary to keep the solution warm to prevent precipitation of the product. The aqueous phase was removed and the organic phase was washed with two 50 ml portions of 5% acetic acid and three 50 ml portions of 5% sodium bicarbonate. The warm organic solution was dried with anhydrous magnesium sulfate. The desiccant was filtered and the solvent was stripped using an aspirator and high vacuum systems. The solid residue was recrystallized from absolute ethanol, yielding 3.8g of pink crystals. The yield of product was 76% and the melting range was 138 to 140°C. The infrared spectrum of this compound showed multiple ketone carbonyl bands around 1620 cm⁻¹ and the hydrazide carbonyl at 1740 cm⁻¹. Structural integrity was further confirmed using proton and carbon-13 NMR spectroscopy.

### EXAMPLE X

### Preparation of (4-benzoyl-3-hydroxyphenoxy)-N'-methacryloyl)acetylhydrazide

The hydrazide Ia (2.9g, 0.01 mole), pyridine (0.95g, 0.012 mole) and 90 ml of tetrahydrofuran were combined in a dried 125 ml flask under a nitrogen atmosphere. An addition funnel was attached and charged with methacryloyl chloride (1.15g, 0.01 mole) and 10 ml of tetrahydrofuran. The acid chloride solution was added dropwise maintaining the reaction temperature about 10°C. The reaction mixture was then stirred at ambient temperature for 4 hours. The reaction mixture was poured into a beaker containing 700 ml of water and stirred for 15 minutes. The product which precipitated was isolated by filtration and subsequently dissolved in 700 ml of warm chloroform for drying with anhydrous magnesium sulfate. The desiccant was removed by filtration and the solvent was stripped using an aspirator and high vacuum systems. The solid was recrystallized from toluene to give 2.4g of product. The yield was 69% of product and the melting range was 153-155°C. The infrared spectrum of this compound showed multiple ketone carbonyls around 1620 cm⁻¹ and merged hydrazide carbonyl absorptions at 1650-1700 cm⁻¹.

### EXAMPLE XI

### Preparation of Ethyl N'-((4-Benzoyl-3-hydroxyphenoxy)acetyl) hydrazinecarboxylate

The hydrazide Ia (4.0g, 0.014 mole), triethylamine (1.5g, 0.015 mole) and 175 ml of tetrahydrofuran (THF) were combined in a dried 500 ml flask under a nitrogen atmosphere. An addition funnel was attached and charged with ethyl chloroformate (1.6g, 0.015 mole) and 25 ml of tetrahydrofuran. The reaction mixture was warmed to 45°C for the slow addition of the chloroformate. After 45 minutes, additional chloroformate (0.8g, 0.007 mole) in 20 ml of THF was added and the resulting mixture was maintained at 45°C for 1 hour. Solid sodium chloride was added and the liquid was decanted in a separatory funnel containing 150 ml of methyl t-butyl ether. The organic solution was washed with two 50 ml portions of saturated sodium bicarbonate. The ether solution was dried using anhydrous magnesium sulfate. The desiccant was filtered and the solvents were removed using an aspirator and high vacuum systems. The product was 4.3g of soft crystals. The yield was 86%. The infrared spectrum of this compound showed multiple ketone carbonyl bands around 1610 cm⁻¹ and the acyl hydrazine carboxylate carbonyls at 1710 and 1760 cm⁻¹.

### EXAMPLE XII

### Reaction of Ia with Maleic Anhydride

Maleic anhydride (2.5g, 0.025 mole) and 150 ml of toluene were combined in a dried 250 ml flask under a nitrogen atmosphere. A catalytic amount of p-toluenesulfonic acid monohydrate (2-3mg) was added. The resulting mixture was refluxed for 30 minutes. The reaction mixture was cooled to ambient temperature and hydrazide Ia (5.7g, 0.02 mole) was added. The resulting reaction mixture was stirred at room temperature for 17 hours and then warmed in a 75°C oil bath for 1 hour. The mixture was cooled to room temperature and diluted with 150 ml of methyl t-butyl ether. The solid was filtered and mixed with 100 ml of pure methyl t-butyl ether. After stirring and warming this mixture for about 15 minutes, it was cooled to room temperature and the solid was isolated by filtration. Residual solvent was removed using a high vacuum system. The resulting pale yellow product weighed 7.2g and melted at 144-149°C. The infrared spectrum of this product showed multiple ketone carbonyls at 1620 cm⁻¹ and a broad band for the remaining merged carbonyls at 1710 cm⁻¹. The material was shown to be > 90% pure by liquid chromatography. The major impurity was starting hydrazide.

### EXAMPLE XIII

### Preparation of N-(4-Hydroxy-3,5-di-t-butylbenzoyl-2-(4-benzoyl-3-hydroxyphenoxy)acetyl hydrazide

Into a 1 flask equipped with a thermometer, magnetic stir bar, addition funnel, and reflux condenser were placed the hydrazide Ia (8.6g, 0.03 mole), 4-hydroxy-3,5-di-t-butylbenzoyl chloride (8.1g, 0.03 mole) and 700 ml of tetrahydrofuran. Triethylamine (3.1g, 0.031 mole) and 10 ml of tetrahydrofuran were mixed and charged to the addition funnel. The amine was added to the reaction mixture over about 10 minutes at a temperature of 24-27°C, accompanied by a mild exotherm and precipitation of triethylamine hydrochloride. The resulting mixture was stirred at 27°C for 2 hours. The reaction was heated to 40-50°C for 1 hour then cooled and filtered free of salt. An additional 1 ml of triethylamine was added to the filtered solution and the resulting mixture was allowed to stand overnight at room temperature. The solvent was stripped using aspirator vacuum to give a yellow solid weighing 16.1g. The solid was dissolved in 30 ml of methylene chloride and washed with 50 ml of 5% aqueous HCl, aqueous sodium carbonate and sodium bicarbonate; then it was dried using anhydrous sodium sulfate. The desiccant was filtered and the solvent was stripped using aspirator vacuum and high vacuum. The resulting product was a yellow powder weighing 11.1g and melting at 108-112°C. The infrared spectrum of this compound showed multiple merged carbonyl bands, the strongest at 1635 cm⁻¹ and the phenolic OH at 3640 cm⁻¹.

### EXAMPLE XIV

### Preparation of diethyl 2-hydroxybenzophenone-4,4'-diyloxy-bis(acetate)

Into a 250 ml round bottom flask equipped with a reflux condenser and mechanical stirrer were placed 2,4,4'-trihydroxybenzophenone (6.9g, 0.03 mole), ethyl chloroacetate (7.4g, 0.06 mole), potassium carbonate (12.4g, 0.09 mole), and 100 ml of methyl ethyl ketone. This mixture was heated to reflux and the reflux was maintained for 5.5 hours. The reaction mixture was cooled to room temperature and was transferred to a separatory funnel, with 300 ml of water, 50 ml of 10% hydrochloric acid, and 300 ml of methyl t-butyl ether. The mixture was shaken, and the phases were allowed to separate. The aqueous phases were drawn off and the organic phases were washed with three 100 ml portions of water and dried with anhydrous magnesium sulfate. The desiccant was filtered and the solvent was stripped (aspirator vacuum) to yield 10.8g of orange crystals. This product was recrystallized from 75 ml of 95% ethanol to give orange crystals which were dried under high vacuum. The yield of purified material was 6.2g of product (51.2% of theory) melting at 99-103°C. The identity of this product was confirmed by NMR spectroscopy.

### B. Preparation of bis hydrazide

The ester from the above experiment (5.9g, 0.015 mole) and 85% hydrazine hydrate (18 ml) were combined with 75 ml of isopropyl alcohol in a 125 ml flask equipped with a magnetic stir bar and nitrogen atmosphere. The mixture was stirred at ambient temperature for 3.5 hours. The mixture was then poured into 1 liter of water and was stirred while acetic acid (75.6g) was added. A very fine crystalline material separated from the solution and was isolated by filtration. The product was air dried on the filtration apparatus for 2 days. The product was 5.3g of orange crystals melting about 232°C. The IR spectrum showed multiple ketone carbonyls at 1610 cm⁻¹ and hydrazide carbonyls at 1660 cm⁻¹. Elemental analysis: calculated: 54.54% C, 4.85% H, 14.97% N, 25.64% O; found: 52.94% C, 5.11% H, 14.46% N, 27.11% O.

### EXAMPLE XV

### Preparation of 2-(4-Benzoyl-3-hydroxyphenoxy)ethyl carbazate

Into a 125 ml flask equipped with magnetic stir bar, nitrogen atmosphere, and addition funnel were placed 25 ml of tetrahydrofuran and 3 ml of 85% hydrazine hydrate. The mixture was cooled to 10°C using an ice bath. A solution of 2-(4-benzoyl-3-hydroxyphenoxy)ethyl chloroformate (95.4% assay, 3.4g, 0.01 mole) in 25 ml of tetrahydrofuran was charged into the addition funnel and added dropwise to the hydrazine solution over a 10 minute period, keeping the temperature about 10°C. After addition, the solution was allowed to warm to room temperature and to stir for 4 hours. The reaction mixture was poured into 400 ml of water. An oil separated which slowly solidified. The solid was isolated by filtration and dried in chloroform solution using anhydrous magnesium sulfate. The desiccant was filtered and the solvent was stripped using aspirator vacuum and high vacuum to give a 3.0g of sticky yellow crystals. These crystals were slurried with 15 ml of methyl t-butyl ether to give 1.3g of soft yellow crystals which melted at 85-92°C. The IR spectrum showed multiple ketone carbonyls around 1620 cm⁻¹ and the carbazate carbonyl at 1730 cm⁻¹.

### EXAMPLE XVI

### Preparation of Octylphenoxypoly(ethoxy)ethyl N'-((4-benzoyl-3-hydroxyphenoxy)-acetyl)-hydrazinecarboxylate

Into a 250 ml round bottom flask equipped with a reflux condenser, addition funnel, magnetic stirrer, and nitrogen atmosphere were placed hydrazide Ia (2.0g, 0.007 mole), triethylamine (0.8g, 0.008 mole) and 75 ml of tetrahydrofuran. The addition funnel was charged with octylphenoxy-poly(ethoxy)ethyl chloroformate (5.0g, 0.007 mole by hydrolyzable chloride content, prepared from Triton X-100 (Rohm & Haas) and phosgene) and 25 ml of tetrahydrofuran. The chloroformate was added to the reaction mixture with a slight exotherm noted. After complete addition, the mixture was heated to reflux and refluxed for 2.5 hours. The reaction mixture was cooled and the small amount of solid in the solution was removed by filtration. The solvent was stripped (aspirator vacuum) to yield a residue consisting of a solid in a viscous liquid. This was taken up in 25 ml of acetone and the insoluble solid was filtered. The acetone was stripped (aspirator and high vacuum). The IR spectrum of this material showed 3 carbonyl absorptions at 1770, 1710 and 1600 cm⁻¹. The product was a viscous yellow oil weighing 5.7g (85% of theory).

### EXAMPLE XVII

### ACCELERATED WEATHERING TEST

### A. Resin Formulation

A low molecular weight hydroxy-functional acrylic resin, or oligomer, was prepared by free radical solution polymerization. The polymerization was conducted under nitrogen in a jacketed glass reactor equipped with a stirrer, thermometer, and reflux condenser. The monomers and initiator (listed below) were combined and metered into the reactor containing ethyl 3-ethoxypropionate solvent (150g) at 145°C over a five hour period. After the monomer/initiator addition was completed, polymerization was continued for an additional hour to reduce residual monomers.

| | | Amount |
|---|---|---|
| Monomers: | butyl acrylate | 180g |
| | butyl methacrylate | 120g |
| | 2-hydroxyethyl acrylate | 150g |
| | methyl methacrylate | 72g |
| | styrene | 60g |
| | methacrylic acid | 18g |
| Initiator: | Lupersol 533 M75 (ethyl 3,3-di-(t-amylperoxy)butyrate, 75% in odorless mineral spirits) | |

### B. Sample preparation

A clear acrylic coating formulation comprised the acrylic resin prepared above, melamine crosslinking resin, acid catalyst, and reducing solvents. Coatings were applied by brush onto untreated aluminum panels and cured at 140°C for 20 minutes. Dry film thickness was typically 1.5-2.0 mil. The coating formulation was prepared with and without inclusion of stabilizer Ia.

| | | Amount |
|---|---|---|
| Coating: | Resin (A above) | 20.0g |
| | Cymel 303 (melamine formaldehyde) | 5.0g |
| | n-Butanol | 2.7g |
| | Aromatic-100 (mixed aromatic hydrocarbons) | 2.7g |
| | DBE Solvent (dibasic esters) | 2.7g |
| | Cycat 4040 (40% wt solution of p-toluene sulfonic acid in isopropanol) | 0.2g |
| | (Stabilizer Ia | 0.2g) |

### C. Accelerated Weathering

Cured coating were aged at room temperature for 24 hours, after which gloss (60°C), pencil hardness and solvent resistance (methyl ethyl ketone rubs) were determined. Stabilized and unstabilized coating gave similar results in these tests. Accelerated weathering was conducted with a Q-U-V weathering instrument using 8 hour light cycle (UV-B) at 60°C, and a 4 hour wet cycle at 50°C. Exposed panels were inspected at regular intervals for loss in gloss. This was determined by the average of three panel measurements. The test results are shown in the following table and demonstrate the effectiveness of the stabilizer Ia for retaining initial coating properties.

**TABLE I**

| Accelerated Weathering Results | | | | | |
|---|---|---|---|---|---|
| Stabilizer | % gloss retention after exposure (hours) | | | | |
| | 0 | 750 | 1000 | 1200 | 1450 |
| Ia | 100 | 98 | 91 | 54 | 46 |
| none | 100 | 76 | 60 | 51 | (failed) |

## Claims (Claims for the following Contracting State(s): BE, CH, DE, FR, GB, IT, LI, NL, SE)

1. A compound of formula (I) 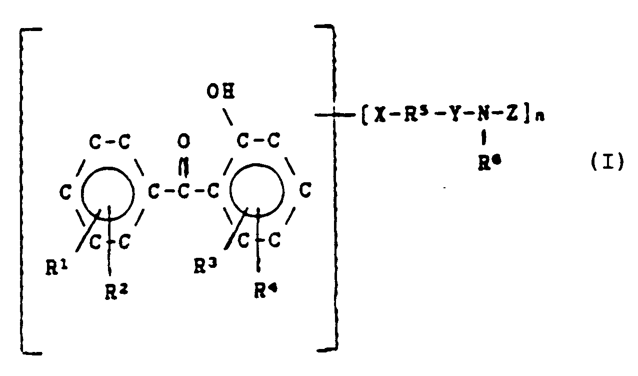 wherein
R¹, R², R³ and R⁴ are independently selected from hydrogen, substituted or unsubstituted alkyl of 1 to 10 carbons, substituted or unsubstituted acyl of 1 to 10 carbons, substituted or unsubstituted alkoxy of 1 to 18 carbons, substituted or unsubstituted aryl of 6 to 10 carbons, substituted or unsubstituted aralkyl of 7 to 16 carbons, substituted or unsubstituted alkoxycarbonyl of 2 to 19 carbons, substituted or unsubstituted aryloxy of 6 to 10 carbons, substituted or unsubstituted alkylmercapto of 1 to 10 carbons, substituted or unsubstituted arylmercapto of 6 to 10 carbons, substituted or unsubstituted alkylamino of 1 to 5 carbons, substituted or unsubstituted dialkylamino of 2 to 10 carbons, substituted or unsubstituted arylamino of 6 to 10 carbons, substituted or unsubstituted arylalkylamino of 7 to 20 carbons, substituted or unsubstituted cycloalkyl of 3 to 12 carbons, substituted or unsubstituted aroyl of 7 to 11 carbons, carboxy, hydroxy, chloro, bromo, cyano, carbamyl, sulfamyl, alkylcarbamyl of 1 to 10 carbons, alkyl sulfamyl of 1 to 10 carbons, alkoxycarbonyloxy of 2 to 11 carbons, acyloxy of 1 to 10 carbons, wherein the substituents are selected from chloro, bromo, cyano, nitro, mercapto, alkylmercapto of 1 to 4 carbons, alkoxy of 1 to 4 carbons, hydroxy, carboxy, carbonyl, alkoxy carbonyl of 2 to 12 total carbons, and carboxylate,
X is -O-, -N(R⁶)-, -S-, -O-C(=O)- or a direct bond between the aromatic ring and R⁵,
R⁵ is selected from a direct bond or alkylene of 1 to 10 carbons, alkenylene of 2 to 10 carbons, 1,4 or 1,3 or 1,2-phenylene, or cycloalkanediyl of 5 to 12 carbons, and optional substituents for said diradicals are selected from cyano, chloro, bromo, alkyl of 1 to 4 carbons, acyl of 1 to 4 carbons, alkoxy of 1 to 4 carbons, carboxy, alkoxycarbonyl of 2 to 6 carbons, alkoxyalkyl of 2 to 6 carbons, or alkoxyalkenoxy of 3 to 6 carbons,
Y is selected from -C(=O)-, -S(=O)₂-, and -OC(=O)-,
R⁶ is selected from hydrogen, alkyl of 1 to 10 carbons, aryl of 6 to 10 carbons, acyl of 1 to 10 carbons, or aroyl of 7 to 11 carbons,
Z is selected from -N(Q-R⁷)(R⁸), -N=C(R⁹)(R¹⁰), 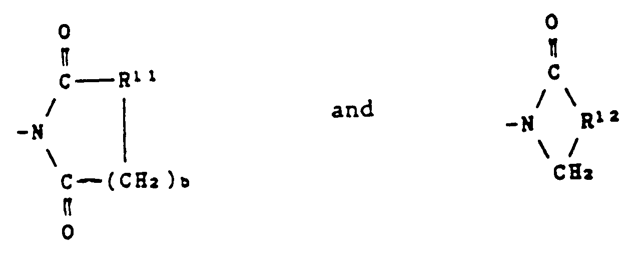 Q is selected from -C(=O)-, -S(=O)₂-, -C(=O)-O-, -C(=O)-N(R⁸)-, and a direct bond between the nitrogen and R⁷, and
R⁷ and R⁸ are independently selected from hydrogen, alkyl of 1 to 20 carbons, substituted or unsubstituted alkenyl of 2 to 6 carbons, aralky of 7 to 12 carbons, cycloalkyl of 5 to 12 carbons, substituted or unsubstituted aryl of 6 to 14 carbons, and polyoxyalkylene of formula 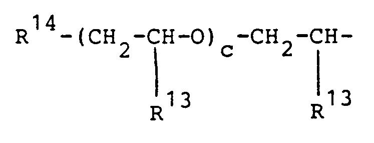 wherein R¹⁴ is selected from alkoxy of 1-20 carbons, aryloxy of 6-10 carbons, alkoxyalkoxy of 3 to 20 carbons, alkyl mercapto of 1-6 carbons and alkenyloxy of 3-7 carbons,
R¹³ is selected from methyl and hydrogen, and
c is an integer of 2 to 50, and polyalkyl of the structure
CH₃-(CH₂)_{d}-
in which d is an integer of 25 to 50, and substituted triazines of structure 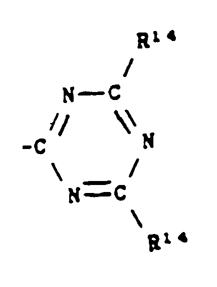 wherein R¹⁴ is as previously defined,
b is O or 1,
R⁹ and R¹⁰ are independently selected from hydrogen, alkyl of 1 to 10 carbons, aryl of 6 to 10 carbons, and R⁹ and R¹⁰ may be joined to form a substituted or unsubstituted ring of 5 to 12 carbons which may optionally contain unsaturation and heteroatoms selected from nitrogen, oxygen and sulfur,
R¹¹ and R¹² are independently selected from alkylene of 2 to 5 carbons, alkenylene of 2 to 3 carbons, aryl diradical of 6 to 12 carbons, and aralkyl diradical of 7 to 15 carbons wherein the alkylene and alkenylene diradicals may optionally contain heteroatoms selected from nitrogen, oxygen and sulfur,
wherein R⁷, R⁸, R⁹, R¹⁰ and R¹¹ may optionally be substituted by one or more hydroxy, chloro, bromo, cyano, nitro, carboxy, alkoxy of 1 to 4 carbons, alkoxycarbonyl of 2 to 6 carbons, and alkyl of 1 to 4 carbons, and
n is 1 or 2.

2. The compound of Claim 1 in which R¹, R², R³,
and R⁴ are independently selected from
hydrogen, substituted or unsubstituted alkyl of 1 to 4 carbons, substituted or unsubstituted alkoxy of 1 to 12 carbons, substituted or unsubstituted aryl of 6 carbons, substituted or unsubstituted aralkyl of 7 to 9 carbons, carboxy, hydroxy, or chloro,
X is selected from -O-, -O-C(=O)-, and a direct bond between the ring and R⁵,
R⁵ is selected from a direct bond or substituted or unsubstituted alkylene of 1 to 8 carbons, substituted or unsubstituted alkenylene of 2 to 6 carbons, or substituted or unsubstituted 1,2 or 1,3 or 1,4 phenylene,
R⁶ is selected from hydrogen, alkyl of 1 to 4 carbons, acyl of 1 to 6 carbons, and aroyl of 7 to 9 carbons,
Z is selected from -N(Q-R⁷) (R⁸), -N=C(R⁹) (R¹⁰), and 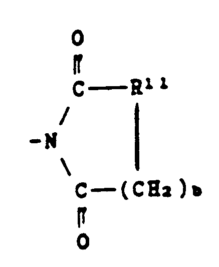 Q is selected from -C(=O)-, -C(=O)-O-, -C(=O)-N(R⁸)-, and a direct bond between the nitrogen and R⁷,
R⁷ is selected from hydrogen, alkyl of 1 to 8 carbons, polyoxyalkylene, substituted or unsubstituted alkenyl of 2 to 6 carbons, and substituted or unsubstituted aryl of 6 to 14 carbons,
R⁸ is hydrogen, alkyl of 1 to 8 carbons, or aryl of 6 to 10 carbons,
b is O,
R⁹ and R¹⁰ are selected from hydrogen, substituted or unsubstituted alkyl of 1 to 8 carbons, and may be joined to form a substituted or unsubstituted ring of 5 to 7 carbons which may optionally contain unsaturation and heteroatoms selected from nitrogen, oxygen and sulfur, and
R¹¹ and R¹² are selected from alkylene of 2 to 5 carbons, alkenylene of 2 to 3 carbons, and aryl diradical of 6 to 12 carbons.

3. The compound of Claim 2 in which R¹, R², R³, and R⁴ are selected from hydrogen, substituted or unsubstituted alkoxy of 1 to 4 carbons, hydroxy, or chloro,
X is -O- or a direct bond between the ring and R⁵,
R⁵ is a direct bond or substituted or unsubstituted alkylene of 1 to 6 carbons,
R⁶ is hydrogen, alkyl of 1-4 carbons, or acyl of 1-4 carbons,
Q is -C(=O)-, -C(=O)-O-, and a direct bond between the nitrogen and R⁷, and
R⁷ is selected from hydrogen, alkyl of 1 to 4 carbons, polyoxyethylene, or substituted or unsubstituted aryl of 6 to 14 carbons,
R⁸ is hydrogen, alkyl of 1 to 4 carbons, or aryl of 6 carbons, and
R¹¹ and R¹² are independently selected from alkylene of 2 to 3 carbons, alkenylene of 2 carbons, and arylene of 6-10 carbons.

4. The compound of Claim 3 is (4-benzoyl-3-hydroxyphenoxy)acetyl hydrazide.

5. The compound of Claim 3 is 2-(2-hydroxybenzoyl)-benzoic acid hydrazide.

6. The compound of Claim 3 is 2-hydroxybenzophenone-4,4'-diyloxy bis(acetyl hydrazide).

7. The compound of Claim 3 is 2,2'-dihydroxybenzophenone-4,4'-diyloxy bis(acetyl hydrazide).

8. The compound of Claim 3 is 2,2,6,6-tetramethyl-4-piperidone, 4-benzoyl-3-hydroxyphenoxyacetyl hydrazide hydrazone or N-[(4-benzoyl-3-hydroxyphenoxy)-acetyl]-N'-(3,5-di-t-butyl-4-hydroxybenzoyl)hydrazine or 2-(4-benzoyl-3-hydroxyphenoxy)ethyl N'[(4-benzoyl-3-hydroxyphenoxy)acetyl]hydrazine carboxylate.

9. Use of compounds of one of Claims 1 to 8 for stabilizing polymers and copolymers.

10. Composition on the basis of at least one polymer or copolymer, characterized by a content of at least one compound of claims 1 to 8.

## Claims (Claims for the following Contracting State(s): ES)

1. A process of preparing a compound of the formula 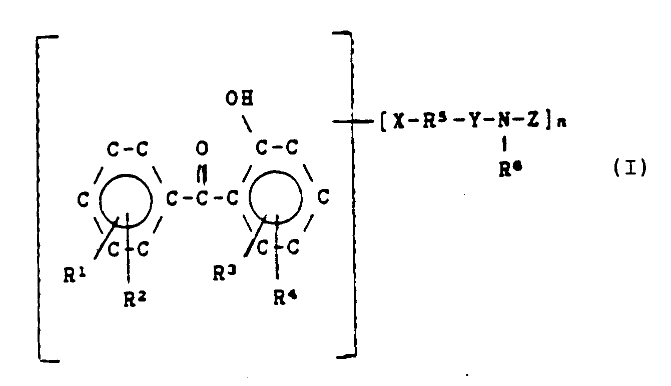 wherein
R¹, R², R³ and R⁴ are independently selected from hydrogen, substituted or unsubstituted alkyl of 1 to 10 carbons, substituted or unsubstituted acyl of 1 to 10 carbons, substituted or unsubstituted alkoxy of 1 to 18 carbons, substituted or unsubstituted aryl of 6 to 10 carbons, substituted or unsubstituted aralkyl of 7 to 16 carbons, substituted or unsubstituted alkoxycarbonyl of 2 to 19 carbons, substituted or unsubstituted aryloxy of 6 to 10 carbons, substituted or unsubstituted alkylmercapto of 1 to 10 carbons, substituted or unsubstituted arylmercapto of 6 to 10 carbons, substituted or unsubstituted alkylamino of 1 to 5 carbons, substituted or unsubstituted dialkylamino of 2 to 10 carbons, substituted or unsubstituted arylamino of 6 to 10 carbons, substituted or unsubstituted arylalkylamino of 7 to 20 carbons, substituted or unsubstituted cycloalkyl of 3 to 12 carbons, substituted or unsubstituted aroyl of 7 to 11 carbons, carboxy, hydroxy, chloro, bromo, cyano, carbamyl, sulfamyl, alkylcarbamyl or 1 to 10 carbons, alkyl sulfamyl of 1 to 10 carbons, alkoxycarbonyloxy of 2 to 11 carbons, acyloxy of 1 to 10 carbons, wherein the substituents are selected from chloro, bromo, cyano, nitro, mercapto, alkylmercapto of 1 to 4 carbons, alkoxy of 1 to 4 carbons, hydroxy, carboxy, carbonyl, alkoxy carbonyl of 2 to 12 total carbons, and carboxylate,
X is -O-, -N(R⁶)-, -S-, -O-C(=O)- or a direct bond between the aromatic ring and R⁵,
R⁵ is selected from a direct bond or alkylene of 1 to 10 carbons, alkenylene of 2 to 10 carbons, 1,4 or 1,3 or 1,2-phenylene, or cycloalkanediyl of 5 to 12 carbons, and optional substituents for said diradicals are selected from cyano, chloro, bromo, alkyl of 1 to 4 carbons, acyl of 1 to 4 carbons, alkoxy of 1 to 4 carbons, carboxy, alkoxycarbonyl of 2 to 6 carbons, alkoxyalkyl of 2 to 6 carbons, or alkoxyalkenoxy of 3 to 6 carbons,
Y is selected from -C(=O)-, -S(=O)₂-, and -OC(=O)-,
R⁶ is selected from hydrogen, alkyl of 1 to 10 carbons, aryl of 6 to 10 carbons, acyl of 1 to 10 carbons, or aroyl of 7 to 11 carbons,
Z is selected from -N(Q-R⁷)(R⁸), -N=C(R⁹)(R¹⁰), 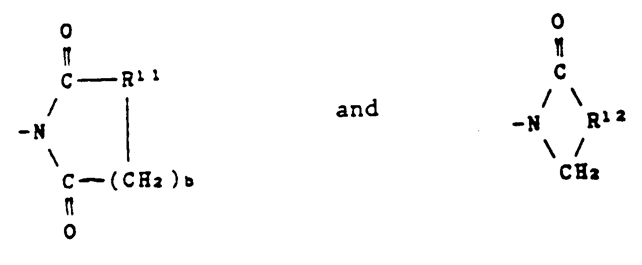 Q is selected from -C(=O)-, -S(=O)₂-, -C(=O)-O-, -C(=O)-N(R⁸)-, and a direct bond between the nitrogen and R⁷, and
R⁷ and R⁸ are independently selected from hydrogen, alkyl of 1 to 20 carbons, substituted or unsubstituted alkenyl of 2 to 6 carbons, aralkyl of 7 to 12 carbons, cycloalkyl of 5 to 12 carbons, substituted or unsubstituted aryl of 6 to 14 carbons, and polyoxyalkylene of formula 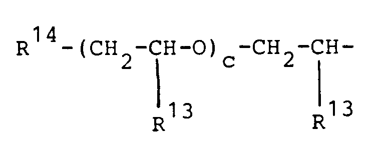 wherein R¹⁴ is selected from alkoxy of 1-20 carbons, aryloxy of 6-10 carbons, alkoxyalkoxy of 3 to 20 carbons, alkyl mercapto of 1-6 carbons and alkenyloxy of 3-7 carbons,
R¹³ is selected from methyl and hydrogen, and
c is an integer of 2 to 50, and polyalkyl of the structure
CH₃-(CH₂)_{d}-
in which d is an integer of 25 to 50, and substituted triazines of structure 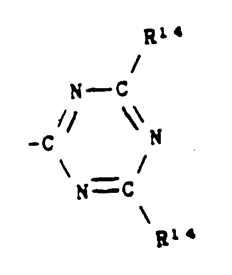 wherein R¹⁴ is as previously defined,
b is O or 1,
R⁹ and R¹⁰ are independently selected from hydrogen, alkyl of 1 to 10 carbons, aryl of 6 to 10 carbons, and R⁹ and R¹⁰ may be joined to form a substituted or unsubstituted ring of 5 to 12 carbons which may optionally contain unsaturation and heteroatoms selected from nitrogen, oxygen and sulfur,
R¹¹ and R¹² are independently selected from alkylene of 2 to 5 carbons, alkenylene of 2 to 3 carbons, aryl diradical of 6 to 12 carbons, and aralkyl diradical of 7 to 15 carbons wherein the alkylene and alkenylene diradicals may optionally contain heteroatoms selected from nitrogen, oxygen and sulfur,
wherein R⁷, R⁸, R⁹, R¹⁰ and R¹¹ may optionally be substituted by one or more hydroxy, chloro, bromo, cyano, nitro, carboxy, alkoxy of 1 to 4 carbons, alkoxycarbonyl of 2 to 6 carbons, and alkyl of 1 to 4 carbons, and
n is 1 or 2,
by reacting a compound of the formula 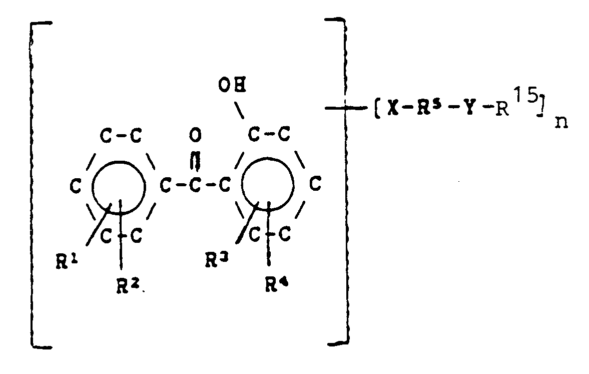 wherein R¹-R⁵, X, Y and n are as previously defined and R¹⁵ is H, chloro or lower alkyl of 1 to 6 carbons with hydrazine or an alkyl or aryl hydrazine to form a compound of formula (I) which may be converted in a manner known per se to other products of formula (I).

2. The process of Claim 1 for preparing a compound in which R¹, R², R³ and R⁴ are independently selected from hydrogen, substituted or unsubstituted alkyl of 1 to 4 carbons, substituted or unsubstituted alkoxy of 1 to 12 carbons, substituted or unsubstituted aryl of 6 carbons, substituted or unsubstituted aralkyl of 7 to 9 carbons, carboxy, hydroxy, or chloro,
X is selected from -O-, -O-C(=O)-, and a direct bond between the ring and R⁵,
R⁵ is selected from a direct bond or substituted or unsubstituted alkylene of 1 to 8 carbons, substituted or unsubstituted alkenylene of 2 to 6 carbons, or substituted or unsubstituted 1,2 or 1,3 or 1,4 phenylene,
R⁶ is selected from hydrogen, alkyl of 1 to 4 carbons, acyl of 1 to 6 carbons, and aroyl of 7 to 9 carbons,
Z is selected from -N(Q-R⁷)(R⁸), -N=C(R⁹)(R¹⁰), and 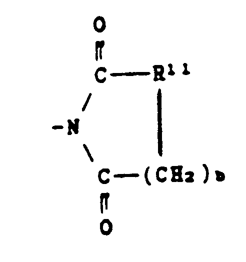 Q is selected from -C(=O)-, -C(=O)-O-, -C(=O)-N(R⁸)-, and a direct bond between the nitrogen and R⁷,
R⁷ is selected from hydrogen, alkyl of 1 to 8 carbons, polyoxyalkylene, substituted or unsubstituted alkenyl of 2 to 6 carbons, and substituted or unsubstituted aryl of 6 to 14 carbons,
R⁸ is hydrogen, alkyl of 1 to 8 carbons, or aryl of 6 to 10 carbons,
b is O,
R⁹ and R¹⁰ are selected from hydrogen, substituted or unsubstituted alkyl of 1 to 8 carbons, and may be joined to form a substituted or unsubstituted ring of 5 to 7 carbons which may optionally contain unsaturation and heteroatoms selected from nitrogen, oxygen and sulfur, and
R¹¹ and R¹² are selected from alkylene of 2 to 5 carbons, alkenylene of 2 to 3 carbons, and aryl diradical of 6 to 12 carbons.

3. The process of Claim 2 for preparing a compound in which R¹, R², R³, and R⁴ are selected from hydrogen, substituted or unsubstituted alkoxy of 1 to 4 carbons, hydroxy, or chloro,
X is -O- or a direct bond between the ring and R⁵,
R⁵ is a direct bond or substituted or unsubstituted alkylene of 1 to 6 carbons,
R⁶ is hydrogen, alkyl of 1-4 carbons, or acyl of 1-4 carbons,
Q is -C(=O)-, -C(=O)-O-, and a direct bond between the nitrogen and R⁷, and
R⁷ is selected from hydrogen, alkyl of 1 to 4 carbons, polyoxyethylene, or substituted or unsubstituted aryl of 6 to 14 carbons,
R⁸ is hydrogen, alkyl of 1 to 4 carbons, or aryl of 6 carbons, and
R¹¹ and R¹² are independently selected from alkylene of 2 to 3 carbons, alkenylene of 2 carbons, and arylene of 6-10 carbons.

4. The process of Claim 3 for preparing a compound which is (4-benzoyl-3-hydroxyphenoxy)acetyl hydrazide.

5. The process of Claim 3 for preparing a compound which is 2-(2-hydroxybenzoyl)benzoic acid hydrazide.

6. The process of Claim 3 for preparing a compound which is 2-hydroxybenzophenone-4,4'-diyloxy bis(acetyl hydrazide).

7. The process of Claim 3 for preparing a compound which is 2,2'-dihydroxybenzophenone-4,4'-diyloxy bis(acetyl hydrazide).

8. The process of Claim 3 for preparing a compound which is 2,2,6,6-tetramethyl-4-piperidone, 4-benzoyl-3-hydroxyphenoxyacetyl hydrazide hydrazone or N-[(4-benzoyl-3-hydroxyphenoxy)-acetyl]-N'-(3,5-di-t-butyl-4-hydroxybenzoyl)-hydrazine or 2-(4-benzoyl-3-hydroxyphenoxy)ethyl N'[(4-benzoyl-3-hydroxyphenoxy)acetyl]-hydrazine carboxylate.

9. Use of compounds prepared according to one of claims 1 to 8 for stabilizing polymers and copolymers.

10. Composition on the basis of at least one polymer or copolymer, characterized by a content of at least one compound prepared according to one of claims 1 to 8.

## Patentansprüche (Patentansprüche für folgende(n) Vertragsstaat(en): BE, CH, DE, FR, GB, IT, LI, NL, SE)

1. Eine Verbindung der Formel (I) 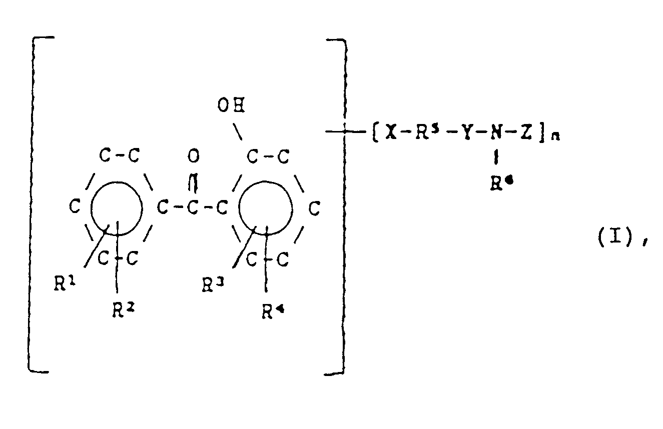 worin
R¹, R², R³ und R⁴ unabhängig voneinander ausgewählt sind aus Wasserstoff, substituiertem oder unsubstituiertem Alkyl mit 1 bis 10 Kohlenstoffatomen, substituiertem oder unsubstituiertem Acyl mit 1 bis 10 Kohlenstoffatomen, substituiertem oder unsubstituiertem Alkoxy mit 1 bis 18 Kohlenstoffatomen, substituiertem oder unsubstituiertem Aryl mit 6 bis 10 Kohlenstoffatomen, substituiertem oder unsubstituiertem Aralkyl mit 7 bis 16 Kohlenstoffatomen, substituiertem oder unsubstituiertem Alkoxycarbonyl mit 2 bis 19 Kohlenstoffatomen, substituiertem oder unsubstituiertem Aryloxy mit 6 bis 10 Kohlenstoffatomen, substituiertem oder unsubstituiertem Alkylmercapto mit 1 bis 10 Kohlenstoffatomen, substituiertem oder unsubstituiertem Arylmercapto mit 6 bis 10 Kohlenstoffatomen, substituiertem oder unsubstituiertem Alkylamino mit 1 bis 5 Kohlenstoffatomen, substituiertem oder unsubstituiertem Dialkylamino mit 2 bis 10 Kohlenstoffatomen, substituiertem oder unsubstituiertem Arylamino mit 6 bis 10 Kohlenstoffatomen, substituiertem oder unsubstituiertem Arylalkylamino mit 7 bis 20 Kohlenstoffatomen, substituiertem oder unsubstituiertem Cycloalkyl mit 3 bis 12 Kohlenstoffatomen, substituiertem oder unsubstituiertem Aroyl mit 7 bis 11 Kohlenstoffatomen, Carboxy, Hydroxy, Chlor, Brom, Cyano, Carbamyl, Sulfamyl, Alkylcarbamyl mit 1 bis 10 Kohlenstoffatomen, Alkylsulfamyl mit 1 bis 10 Kohlenstoffatomen, Alkoxycarbonyloxy mit 2 bis 11 Kohlenstoffatomen, Acyloxy mit 1 bis 10 Kohlenstoffatomen, worin die Substituenten ausgewählt sind aus Chlor, Brom, Cyano, Nitro, Mercapto, Alkylmercapto mit 1 bis 4 Kohlenstoffatomen, Alkoxy mit 1 bis 4 Kohlenstoffatomen, Hydroxy, Carboxy, Carbonyl, Alkoxycarbonyl mit insgesamt 2 bis 12 Kohlenstoffatomen und Carboxylat,
X -O-, -N(R⁶)-, -S-, -O-C(=O)- oder eine direkte Bindung zwischen dem aromatischen Ring und R⁵ ist,
R⁵ ausgewählt ist aus einer direkten Bindung oder Alkylen mit 1 bis 10 Kohlenstoffatomen, Alkenylen mit 2 bis 10 Kohlenstoffatomen, 1,4- oder 1,3- oder 1,2-Phenylen oder Cycloalkandiyl mit 5 bis 12 Kohlenstoffatomen, und gegebenenfalls vorhandene Substituenten für die zweibindigen Reste ausgewählt sind aus Cyano, Chlor, Brom, Alkyl mit 1 bis 4 Kohlenstoffatomen, Acyl mit 1 bis 4 Kohlenstoffatomen, Alkoxy mit 1 bis 4 Kohlenstoffatomen, Carboxy, Alkoxycarbonyl mit 2 bis 6 Kohlenstoffatomen, Alkoxyalkyl mit 2 bis 6 Kohlenstoffatomen oder Alkoxyalkenoxy mit 3 bis 6 Kohlenstoffatomen,
Y ausgewählt ist aus -C(=O)-, S(=O)₂- und -OC(=O)-,
R⁶ ausgewählt ist aus Wasserstoff, Alkyl mit 1 bis 10 Kohlenstoffatomen, Aryl mit 6 bis 10 Kohlenstoffatomen, Acyl mit 1 bis 10 Kohlenstoffatomen oder Aroyl mit 7 bis 11 Kohlenstoffatomen,
Z ausgewählt ist aus -N(Q-R⁷)(R⁸), -N=C(R⁹)(R¹⁰), 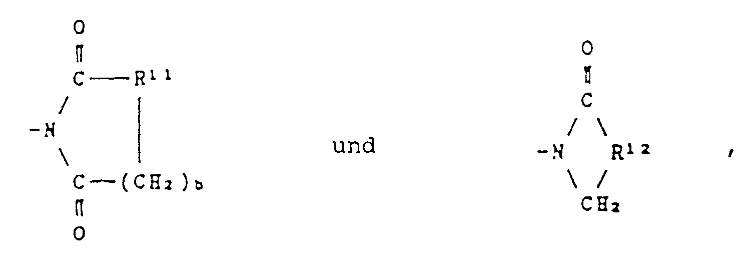 Q ausgewählt ist aus -C(=O)-, -S(=O)₂-, -C(=O)-O-, -C(=O)-N(R⁸)- und einer direkten Bindung zwischen dem Stickstoff und R⁷, und
R⁷ und R⁸ unabhängig voneinander ausgewählt sind aus Wasserstoff, Alkyl mit 1 bis 20 Kohlenstoffatomen, substituiertem oder unsubstituiertem Alkenyl mit 2 bis 6 Kohlenstoffatomen, Aralkyl mit 7 bis 12 Kohlenstoffatomen, Cycloalkyl mit 5 bis 12 Kohlenstoffatomen, substituiertem oder unsubstituiertem Aryl mit 6 bis 14 Kohlenstoffatomen und Polyoxyalkylen der Formel 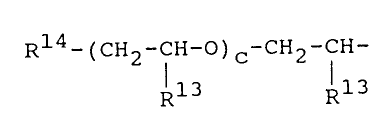 worin R¹⁴ ausgewählt ist aus Alkoxy mit 1-20 Kohlenstoffatomen, Aryloxy mit 6-10 Kohlenstoffatomen, Alkoxyalkoxy mit 3 bis 20 Kohlenstoffatomen, Alkylmercapto mit 1-6 Kohlenstoffatomen und Alkenyloxy mit 3-7 Kohlenstoffatomen,
R¹³ ausgewählt ist aus Methyl und Wasserstoff, und
c eine ganze Zahl von 2 bis 50 ist, und Polyalkyl der Struktur
CH₃-(CH₂)_{d}- ,
in der d eine ganze Zahl von 25 bis 50 ist, und substituierten Triazinen der Struktur 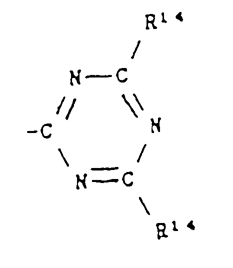 worin R¹⁴ wie oben definiert ist,
b 0 oder 1 ist,
R⁹ und R¹⁰ unabhängig voneinander ausgewählt sind aus Wasserstoff, Alkyl mit 1 bis 10 Kohlenstoffatomen, Aryl mit 6 bis 10 Kohlenstoffatomen, und R⁹ und R¹⁰ unter Bildung eines substituierten oder unsubstituierten Ringes mit 5 bis 12 Kohlenstoffatomen verbunden sein können, der gegebenenfalls Ungesättigtheit und Heteroatome ausgewählt aus Stickstoff, Sauerstoff und Schwefel enthalten kann,
R¹¹ und R¹² unabhängig voneinander ausgewählt sind aus Alkylen mit 2 bis 5 Kohlenstoffatomen, Alkenylen mit 2 bis 3 Kohlenstoffatomen, einem zweibindigen Arylrest mit 6 bis 12 Kohlenstoffatomen und einem zweibindigen Aralkylrest mit 7 bis 15 Kohlenstoffatomen, worin die zweibindigen Alkylen- und Alkenylenreste gegebenenfalls Heteroatome ausgewählt aus Stickstoff, Sauerstoff und Schwefel enthalten können,
wobei R⁷, R⁸, R⁹, R¹⁰ und R¹¹ gegebenenfalls durch ein oder mehrere Hydroxy, Chlor, Brom, Cyano, Nitro, Carboxy, Alkoxy mit 1 bis 4 Kohlenstoffatomen, Alkoxycarbonyl mit 2 bis 6 Kohlenstoffatomen und Alkyl mit 1 bis 4 Kohlenstoffatomen substituiert sein können, und
n 1 oder 2 ist.

2. Die Verbindung nach Anspruch 1, in der R¹, R², R³ und R⁴ unabhängig voneinander ausgewählt sind aus Wasserstoff, substituiertem oder unsubstituiertem Alkyl mit 1 bis 4 Kohlenstoffatomen, substituiertem oder unsubstituiertem Alkoxy mit 1 bis 12 Kohlenstoffatomen, substituiertem oder unsubstituiertem Aryl mit 6 Kohlenstoffatomen, substituiertem oder unsubstituiertem Aralkyl mit 7 bis 9 Kohlenstoffatomen, Carboxy, Hydroxy oder Chlor,
X ausgewählt ist aus -O-, -O-C(=O)- und einer direkten Bindung zwischen dem Ring und R⁵,
R⁵ ausgewählt ist aus einer direkten Bindung oder substituiertem oder unsubstituiertem Alkylen mit 1 bis 8 Kohlenstoffatomen, substituiertem oder unsubstituiertem Alkenylen mit 2 bis 6 Kohlenstoffatomen oder substituiertem oder unsubstituiertem 1,2- oder 1,3- oder 1,4-Phenylen,
R⁶ ausgewählt ist aus Wasserstoff, Alkyl mit 1 bis 4 Kohlenstoffatomen, Acyl mit 1 bis 6 Kohlenstoffatomen und Aroyl mit 7 bis 9 Kohlenstoffatomen,
Z ausgewählt ist aus -N(Q-R⁷)(R⁸), -N=C(R⁹)(R¹⁰) und 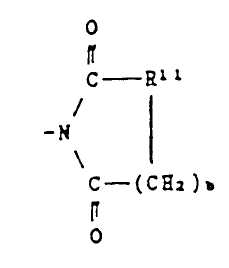 Q ausgewählt ist aus -C(=O)-, -C(=O)-O- -C(=O)-N(R⁸)- und einer direkten Bindung zwischen dem Stickstoff und R⁷,
R⁷ ausgewählt ist aus Wasserstoff, Alkyl mit 1 bis 8 Kohlenstoffatomen, Polyoxyalkylen, substituiertem oder unsubstituiertem Alkenyl mit 2 bis 6 Kohlenstoffatomen und substituiertem oder unsubstituiertem Aryl mit 6 bis 14 Kohlenstoffatomen,
R⁸ Wasserstoff, Alkyl mit 1 bis 8 Kohlenstoffatomen oder Aryl mit 6 bis 10 Kohlenstoffatomen ist,
b O ist,
R⁹ und R¹⁰ ausgewählt sind aus Wasserstoff, substituiertem oder unsubstituiertem Alkyl mit 1 bis 8 Kohlenstoffatomen und unter Bildung eines substituierten oder unsubstituierten Ringes mit 5 bis 7 Kohlenstoffatomen verbunden sein können, der gegebenenfalls Ungesättigtheit und Heteroatome ausgewählt aus Stickstoff, Sauerstoff und Schwefel enthalten kann, und
R¹¹ und R¹² ausgewählt sind aus Alkylen mit 2 bis 5 Kohlenstoffatomen, Alkenylen mit 2 bis 3 Kohlenstoffatomen, und einem zweibindigem Arylrest mit 6 bis 12 Kohlenstoffatomen.

3. Die Verbindung nach Anspruch 2, in der R¹, R², R³ und R⁴ ausgewählt sind aus Wasserstoff, substituiertem oder unsubstituiertem Alkoxy mit 1 bis 4 Kohlenstoffatomen, Hydroxy oder Chlor,
X -O- oder eine direkte Bindung zwischen dem Ring und R⁵ ist,
R⁵ eine direkte Bindung oder substituiertes oder unsubstituiertes Alkylen mit 1 bis 6 Kohlenstoffatomen ist,
R⁶ Wasserstoff, Alkyl mit 1-4 Kohlenstoffatomen oder Acyl mit 1-4 Kohlenstoffatomen ist,
Q -C(=O)-, -C(=O)-O- und eine direkte Bindung zwischen dem Stickstoff und R⁷ ist, und
R⁷ ausgewählt ist aus Wasserstoff, Alkyl mit 1 bis 4 Kohlenstoffatomen, Polyoxyethylen oder substituiertem oder unsubstituiertem Aryl mit 6 bis 14 Kohlenstoffatomen,
R⁸ Wasserstoff, Alkyl mit 1 bis 4 Kohlenstoffatomen oder Aryl mit 6 Kohlenstoffatomen ist, und
R¹¹ und R¹² unabhängig voneinander ausgewählt sind aus Alkylen mit 2 bis 3 Kohlenstoffatomen, Alkenylen mit 2 Kohlenstoffatomen und Arylen mit 6-10 Kohlenstoffatomen.

4. Die Verbindung nach Anspruch 3 ist (4-Benzoyl-3-hydroxyphenoxy)acetylhydrazid.

5. Die Verbindung nach Anspruch 3 ist 2-(2-Hydroxybenzoyl)benzoesäurehydrazid.

6. Die Verbindung nach Anspruch 3 ist 2-Hydroxybenzophenon-4,4'-diyloxy-bis(acetylhydrazid).

7. Die Verbindung nach Anspruch 3 ist 2,2'-Dihydroxybenzophenon-4,4'-diyloxy-bis(acetylhydrazid).

8. Die Verbindung nach Anspruch 3 ist 2,2,6,6-Tetramethyl-4-piperidon, 4-Benzoyl-3-hydroxyphenoxyacetylhyrazidhydrazon oder N-[(4-Benzoyl-3-hydroxyphenoxy)acetyl]-N'-(3,5-di-t-butyl-4-hydroxybenzoyl)hydrazin oder 2-(4-Benzoyl-3-hydroxyphenoxy)ethyl-N'-[(4-benzoyl-3-hydroxyphenoxy)acetyl]hydrazincarboxylat.

9. Verwendung der Verbindungen nach einem der Ansprüche 1 bis 8 zur Stabilisierung von Polymeren und Copolymeren.

10. Zusammensetzung auf der Basis von zumindest einem Polymeren oder Copolymeren, gekennzeichnet durch einen Gehalt an mindestens einer Verbindung der Ansprüche 1 bis 8.

## Patentansprüche (Patentansprüche für folgende(n) Vertragsstaat(en): ES)

1. Ein Verfahren zur Herstellung einer Verbindung der Formel 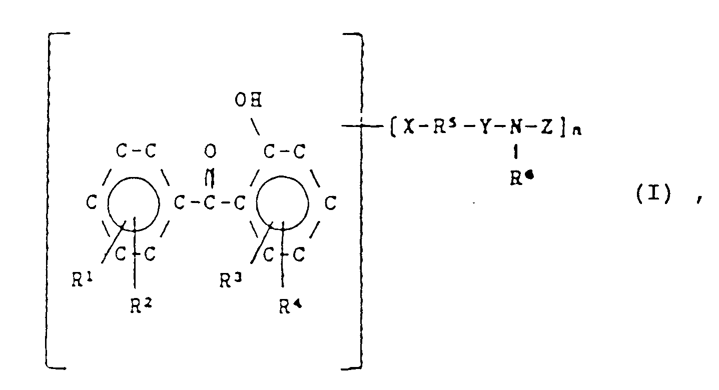 worin
R¹, R², R³ und R⁴ unabhängig voneinander ausgewählt sind aus Wasserstoff, substituiertem oder unsubstituiertem Alkyl mit 1 bis 10 Kohlenstoffatomen, substituiertem oder unsubstituiertem Acyl mit 1 bis 10 Kohlenstoffatomen, substituiertem oder unsubstituiertem Alkoxy mit 1 bis 18 Kohlenstoffatomen, substituiertem oder unsubstituiertem Aryl mit 6 bis 10 Kohlenstoffatomen, substituiertem oder unsubstituiertem Aralkyl mit 7 bis 16 Kohlenstoffatomen, substituiertem oder unsubstituiertem Alkoxycarbonyl mit 2 bis 19 Kohlenstoffatomen, substituiertem oder unsubstituiertem Aryloxy mit 6 bis 10 Kohlenstoffatomen, substituiertem oder unsubstituiertem Alkylmercapto mit 1 bis 10 Kohlenstoffatomen, substituiertem oder unsubstituiertem Arylmercapto mit 6 bis 10 Kohlenstoffatomen, substituiertem oder unsubstituiertem Alkylamino mit 1 bis 5 Kohlenstoffatomen, substituiertem oder unsubstituiertem Dialkylamino mit 2 bis 10 Kohlenstoffatomen, substituiertem oder unsubstituiertem Arylamino mit 6 bis 10 Kohlenstoffatomen, substituiertem oder unsubstituiertem Arylalkylamino mit 7 bis 20 Kohlenstoffatomen, substituiertem oder unsubstituiertem Cycloalkyl mit 3 bis 12 Kohlenstoffatomen, substituiertem oder unsubstituiertem Aroyl mit 7 bis 11 Kohlenstoffatomen, Carboxy, Hydroxy, Chlor, Brom, Cyano, Carbamyl, Sulfamyl, Alkylcarbamyl mit 1 bis 10 Kohlenstoffatomen, Alkylsulfamyl mit 1 bis 10 Kohlenstoffatomen, Alkoxycarbonyloxy mit 2 bis 11 Kohlenstoffatomen, Acyloxy mit 1 bis 10 Kohlenstoffatomen, worin die Substituenten ausgewählt sind aus Chlor, Brom, Cyano, Nitro, Mercapto, Alkylmercapto mit 1 bis 4 Kohlenstoffatomen, Alkoxy mit 1 bis 4 Kohlenstoffatomen, Hydroxy, Carboxy, Carbonyl, Alkoxycarbonyl mit insgesamt 2 bis 12 Kohlenstoffatomen und Carboxylat,
X -O-, -N(R⁶)-, -S-, -O-C(=O)- oder eine direkte Bindung zwischen dem aromatischen Ring und R⁵ ist,
R⁵ ausgewählt ist aus einer direkten Bindung oder Alkylen mit 1 bis 10 Kohlenstoffatomen, Alkenylen mit 2 bis 10 Kohlenstoffatomen, 1,4- oder 1,3- oder 1,2-Phenylen oder Cycloalkandiyl mit 5 bis 12 Kohlenstoffatomen, und gegebenenfalls vorhandene Substituenten für die zweibindigen Reste ausgewählt sind aus Cyano, Chlor, Brom, Alkyl mit 1 bis 4 Kohlenstoffatomen, Acyl mit 1 bis 4 Kohlenstoffatomen, Alkoxy mit 1 bis 4 Kohlenstoffatomen, Carboxy, Alkoxycarbonyl mit 2 bis 6 Kohlenstoffatomen, Alkoxyalkyl mit 2 bis 6 Kohlenstoffatomen oder Alkoxyalkenoxy mit 3 bis 6 Kohlenstoffatomen,
Y ausgewählt ist aus -C(=O)-, -S(=O)₂- und -OC(=O)-,
R⁶ ausgewählt ist aus Wasserstoff, Alkyl mit 1 bis 10 Kohlenstoffatomen, Aryl mit 6 bis 10 Kohlenstoffatomen, Acyl mit 1 bis 10 Kohlenstoffatomen oder Aroyl mit 7 bis 11 Kohlenstoffatomen,
Z ausgewählt ist aus -N(Q-R⁷)(R⁸), -N=C(R⁹)(R¹⁰), 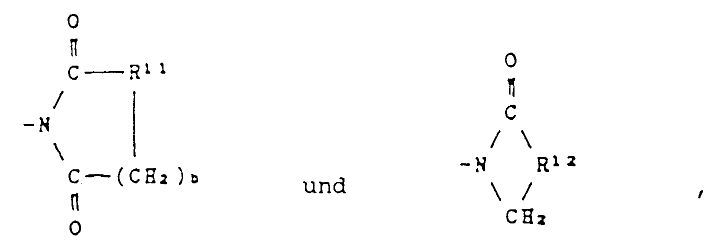 Q ausgewählt ist aus -C(=O)-, -S(=O)₂-, -C(=O)-O-, -C(=O)-N(R⁸)- und einer direkten Bindung zwischen dem Stickstoff und R⁷, und
R⁷ und R⁸ unabhängig voneinander ausgewählt sind aus Wasserstoff, Alkyl mit 1 bis 20 Kohlenstoffatomen, substituiertem oder unsubstituiertem Alkenyl mit 2 bis 6 Kohlenstoffatomen, Aralkyl mit 7 bis 12 Kohlenstoffatomen, Cycloalkyl mit 5 bis 12 Kohlenstoffatomen, substituiertem oder unsubstituiertem Aryl mit 6 bis 14 Kohlenstoffatomen und Polyoxyalkylen der Formel 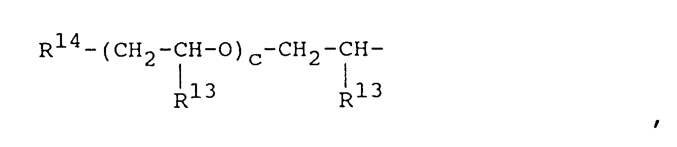 worin R¹⁴ ausgewählt ist aus Alkoxy mit 1-20 Kohlenstoffatomen, Aryloxy mit 6-10 Kohlenstoffatomen, Alkoxyalkoxy mit 3 bis 20 Kohlenstoffatomen, Alkylmercapto mit 1-6 Kohlenstoffatomen und Alkenyloxy mit 3-7 Kohlenstoffatomen,
R¹³ ausgewählt ist aus Methyl und Wasserstoff, und
c eine ganze Zahl von 2 bis 50 ist, und Polyalkyl der Struktur
CH₃-(CH₂)_{d}-,
in der d eine ganze Zahl von 25 bis 50 ist, und substituierten Triazinen der Struktur 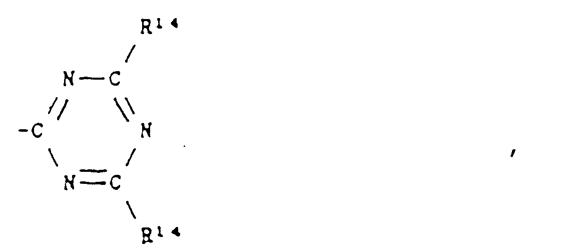 worin R¹⁴ wie oben definiert ist,
b 0 oder 1 ist,
R⁹ und R¹⁰ unabhängig voneinander ausgewählt sind aus Wasserstoff, Alkyl mit 1 bis 10 Kohlenstoffatomen, Aryl mit 6 bis 10 Kohlenstoffatomen, und R⁹ und R¹⁰ unter Bildung eines substituierten oder unsubstituierten Ringes mit 5 bis 12 Kohlenstoffatomen verbunden sein können, der gegebenenfalls Ungesättigtheit und Heteroatome ausgewählt aus Stickstoff, Sauerstoff und Schwefel enthalten kann,
R¹¹ und R¹² unabhängig voneinander ausgewählt sind aus Alkylen mit 2 bis 5 Kohlenstoffatomen, Alkenylen mit 2 bis 3 Kohlenstoffatomen, einem zweibindigen Arylrest mit 6 bis 12 Kohlenstoffatomen und einem zweibindigen Aralkylrest mit 7 bis 15 Kohlenstoffatomen, worin die zweibindigen Alkylen- und Alkenylenreste gegebenenfalls Heteroatome ausgewählt aus Stickstoff, Sauerstoff und Schwefel enthalten können,
wobei R⁷, R⁸, R⁹, R¹⁰ und R¹¹ gegebenenfalls durch ein oder mehrere Hydroxy, Chlor, Brom, Cyano, Nitro, Carboxy, Alkoxy mit 1 bis 4 Kohlenstoffatomen, Alkoxycarbonyl mit 2 bis 6 Kohlenstoffatomen und Alkyl mit 1 bis 4 Kohlenstoffatomen substituiert sein können, und
n 1 oder 2 ist,
durch Reaktion einer Verbindung der Formel 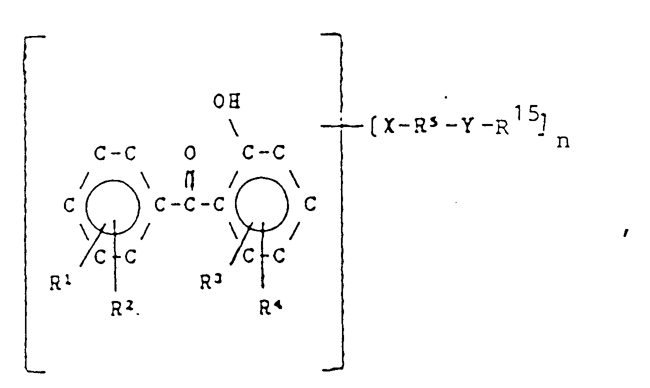 worin R¹-R⁵, X, Y und n wie oben definiert sind, und R¹⁵ H, Chlor oder Niederalkyl mit 1 bis 6 Kohlenstoffatomen ist, mit Hydrazin oder einem Alkyl- oder Arylhydrazin unter Bildung einer Verbindung der Formel (I), die in an sich bekannter Weise in andere Produkte der Formel (I) überführt werden kann.

2. Das Verfahren nach Anspruch 1 zur Herstellung einer Verbindung, in der R¹, R², R³ und R⁴ unabhängig voneinander ausgewählt sind aus Wasserstoff, substituiertem oder unsubstituiertem Alkyl mit 1 bis 4 Kohlenstoffatomen, substituiertem oder unsubstituiertem Alkoxy mit 1 bis 12 Kohlenstoffatomen, substituiertem oder unsubstituiertem Aryl mit 6 Kohlenstoffatomen, substituiertem oder unsubstituiertem Aralkyl mit 7 bis 9 Kohlenstoffatomen, Carboxy, Hydroxy oder Chlor,
X ausgewählt ist aus -O-, -O-C(=O)- und einer direkten Bindung zwischen dem Ring und R⁵,
R⁵ ausgewählt ist aus einer direkten Bindung oder substituiertem oder unsubstituiertem Alkylen mit 1 bis 8 Kohlenstoffatomen, substituiertem oder unsubstituiertem Alkenylen mit 2 bis 6 Kohlenstoffatomen oder substituiertem oder unsubstituiertem 1,2- oder 1,3- oder 1,4-Phenylen,
R⁶ ausgewählt ist aus Wasserstoff, Alkyl mit 1 bis 4 Kohlenstoffatomen, Acyl mit 1 bis 6 Kohlenstoffatomen und Aroyl mit 7 bis 9 Kohlenstoffatomen,
Z ausgewählt ist aus -N(Q-R⁷)(R⁸), -N=C(R⁹)(R¹⁰) und 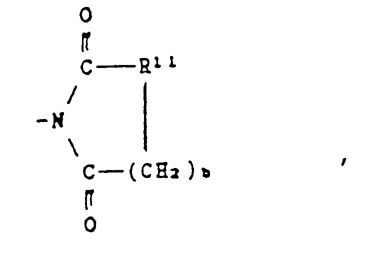 Q ausgewählt ist aus -C(=O)-, -C(=O)-O- -C(=O)-N(R⁸)- und einer direkten Bindung zwischen dem Stickstoff und R⁷,
R⁷ ausgewählt ist aus Wasserstoff, Alkyl mit 1 bis 8 Kohlenstoffatomen, Polyoxyalkylen, substituiertem oder unsubstituiertem Alkenyl mit 2 bis 6 Kohlenstoffatomen und substituiertem oder unsubstituiertem Aryl mit 6 bis 14 Kohlenstoffatomen,
R⁸ Wasserstoff, Alkyl mit 1 bis 8 Kohlenstoffatomen oder Aryl mit 6 bis 10 Kohlenstoffatomen ist,
b O ist,
R⁹ und R¹⁰ ausgewählt sind aus Wasserstoff, substituiertem oder unsubstituiertem Alkyl mit 1 bis 8 Kohlenstoffatomen und unter Bildung eines substituierten oder unsubstituierten Ringes mit 5 bis 7 Kohlenstoffatomen verbunden sein können, der gegebenenfalls Ungesättigtheit und Heteroatome ausgewählt aus Stickstoff, Sauerstoff und Schwefel enthalten kann, und
R¹¹ und R¹² ausgewählt sind aus Alkylen mit 2 bis 5 Kohlenstoffatomen, Alkenylen mit 2 bis 3 Kohlenstoffatomen, und einem zweibindigem Arylrest mit 6 bis 12 Kohlenstoffatomen.

3. Das Verfahren nach Anspruch 2 zur Herstellung einer Verbindung, in der R¹, R², R³ und R⁴ ausgewählt sind aus Wasserstoff, substituiertem oder unsubstituiertem Alkoxy mit 1 bis 4 Kohlenstoffatomen, Hydroxy oder Chlor,
X -O- oder eine direkte Bindung zwischen dem Ring und R⁵ ist,
R⁵ eine direkte Bindung oder substituiertes oder unsubstituiertes Alkylen mit 1 bis 6 Kohlenstoffatomen ist,
R⁶ Wasserstoff, Alkyl mit 1-4 Kohlenstoffatomen oder Acyl mit 1-4 Kohlenstoffatomen ist,
Q -C(=O)-, -C(=O)-O- und eine direkte Bindung zwischen dem Stickstoff und R⁷ ist, und
R⁷ ausgewählt ist aus Wasserstoff, Alkyl mit 1 bis 4 Kohlenstoffatomen, Polyoxyethylen oder substituiertem oder unsubstituiertem Aryl mit 6 bis 14 Kohlenstoffatomen,
R⁸ Wasserstoff, Alkyl mit 1 bis 4 Kohlenstoffatomen oder Aryl mit 6 Kohlenstoffatomen ist, und
R¹¹ und R¹² unabhängig voneinander ausgewählt sind aus Alkylen mit 2 bis 3 Kohlenstoffatomen, Alkenylen mit 2 Kohlenstoffatomen und Arylen mit 6-10 Kohlenstoffatomen.

4. Das Verfahren nach Anspruch 3 zur Herstellung einer Verbindung, die (4-Benzoyl-3-hydroxyphenoxy)acetylhydrazid ist.

5. Das Verfahren nach Anspruch 3 zur Herstellung einer Verbindung, die 2-(2-Hydroxybenzoyl)benzoesäurehydrazid ist.

6. Das Verfahren nach Anspruch 3 zur Herstellung einer Verbindung, die 2-Hydroxybenzophenon-4,4'-diyloxy-bis(acetylhydrazid) ist.

7. Das Verfahren nach Anspruch 3 zur Herstellung einer Verbindung, die 2,2'-Dihydroxybenzophenon-4,4'-diyloxy-bis(acetylhydrazid) ist.

8. Das Verfahren nach Anspruch 3 zur Herstellung einer Verbindung, die 2,2,6,6-Tetramethyl-4-piperidon, 4-Benzoyl-3-hydroxyphenoxyacetylhyrazidhydrazon oder N-[(4-Benzoyl-3-hydroxyphenoxy)-acetyl]-N'-(3,5-di-t-butyl-4-hydroxybenzoyl)hydrazin oder 2-(4-Benzoyl-3-hydroxyphenoxy)ethyl-N'-[(4-benzoyl-3-hydroxyphenoxy)acetyl]hydrazincarboxylat ist.

9. Verwendung der gemäß einem der Ansprüche 1 bis 8 hergestellten Verbindungen zur Stabilisierung von Polymeren und Copolymeren.

10. Zusammensetzung auf der Basis von zumindest einem Polymeren oder Copolymeren, gekennzeichnet durch einen Gehalt an mindestens einer gemäß einem der Ansprüche 1 bis 8 hergestellten Verbindung.

## Revendications (Revendications pour l'(les) Etat(s) contractant(s) suivant(s): BE, CH, DE, FR, GB, IT, LI, NL, SE)

1. Composé de formule (I) 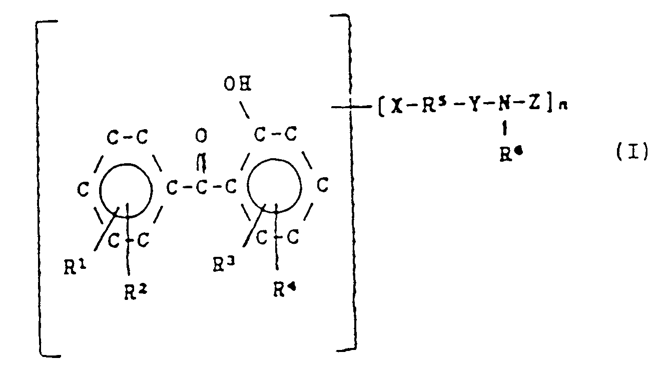 dans laquelle
R¹, R², R³ et R⁴ sont choisis, indépendamment les uns des autres, entre l'hydrogène, un groupe alkyle de 1 à 10 atomes de carbone substitué ou non substitué, un groupe acyle de 1 à 10 atomes de carbone substitué ou non substitué, un groupe alkoxy de 1 à 18 atomes de carbone substitué ou non substitué, un groupe aryle de 6 à 10 atomes de carbone substitué ou non substitué, un groupe aralkyle de 7 à 16 atomes de carbone substitué ou non substitué, un groupe alkoxycarbonyle de 2 à 19 atomes de carbone substitué ou non substitué, un groupe aryloxy de 6 à 10 atomes de carbone substitué ou non substitué, un groupe alkylmercapto de 1 à 10 atomes de carbone substitué ou non substitué, un groupe arylmercapto de 6 à 10 atomes de carbone substitué ou non substitué, un groupe alkylamino de 1 à 5 atomes de carbone substitué ou non substitué, un groupe dialkylamino de 2 à 10 atomes de carbone substitué ou non substitué, un groupe arylamino de 6 à 10 atomes de carbone substitué ou non substitué, un groupe arylalkylamino de 7 à 20 atomes de carbone substitué ou non substitué, un groupe cycloalkyle de 3 à 12 atomes de carbone substitue ou non substitue, un groupe aroyle de 7 à 11 atomes de carbone substitué ou non substitué, un groupe carboxy, hydroxy, chloro, bromo, cyano, carbamyle, sulfamyle, alkylcarbamyle ayant 1 à 10 atomes de carbone, alkylsulfamyle ayant 1 à 10 atomes de carbone, alkoxycarbonyloxy de 2 à 11 atomes de carbone, acyloxy de 1 à 10 atomes de carbone, dont les substituants sont choisis entre des substituants chloro, bromo, cyano, nitro, mercapto, alkylmercapto de 1 à 4 atomes de carbone, alkoxy de 1 à 4 atomes de carbone, hydroxy, carboxy, carbonyle, alkoxycarbonyle de 2 à 12 atomes de carbone au total et carboxylate,
X représente -O-, -N(R⁶)-, -S-, -O-C(=O)- ou une liaison directe entre le noyau aromatique et R⁵,
R⁵ est une liaison directe ou un groupe alkylène de 1 à 10 atomes de carbone, alcénylène de 2 à 10 atomes de carbone, 1,4- ou 1,3- ou 1,2-phénylène, ou cycloalcanediyle de 5 à 12 atomes de carbone, et les substituants facultatifs de ces diradicaux sont choisis entre des substituants cyano, chloro, bromo, alkyle ayant 1 à 4 atomes de carbone, acyle ayant 1 à 4 atomes de carbone, alkoxy ayant 1 à 4 atomes de carbone, carboxy, alkoxycarbonyle de 2 à 6 atomes de carbone, alkoxyalkyle de 2 à 6 atomes de carbone ou alkoxyalcénoxy de 3 à 6 atomes de carbone,
Y est choisi entre -C(=O)-, -S(=O)₂,- et -OC(=O)-,
R⁶ est l'hydrogène, un groupe alkyle de 1 à 10 atomes de carbone, aryle de 6 à 10 atomes de carbone, acyle de 1 à 10 atomes de carbone ou aroyle de 7 à 11 atomes de carbone,
Z est choisi entre les groupes -N(Q-R⁷) (R⁸), -N=C(R⁹)(R¹⁰), 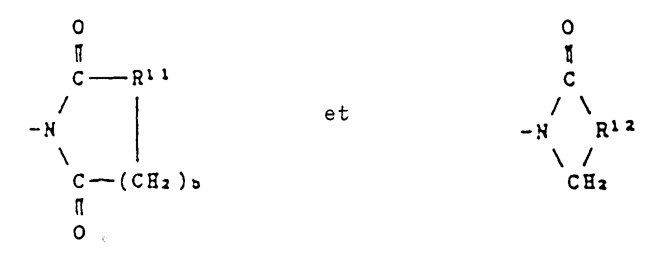 Q est choisi entre -C(=O)-, -S(=O)₂-, -C(=O)-O-, -C(=O)-N(R⁸)- et une liaison directe entre l'azote et R⁷, et
R⁷ et R⁸ sont choisis indépendamment entre l'hydrogène, un groupe alkyle de 1 à 20 atomes de carbone, alcényle de 2 à 6 atomes de carbone substitué ou non substitué, aralkyle de 7 à 12 atomes de carbone, cycloalkyle de 5 à 12 atomes de carbone, aryle de 6 à 14 atomes de carbone substitué ou non substitué et polyoxyalkylène de formule 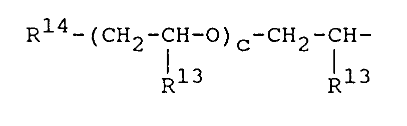 dans laquelle R¹⁴ est choisi entre un groupe alkoxy de 1 à 20 atomes de carbone, aryloxy de 6 à 10 atomes de carbone, alkoxyalkoxy de 3 à 20 atomes de carbone, alkylmercapto de 1 à 6 atomes de carbone et alcényloxy de 3 à 7 atomes de carbone,
R¹³ est choisi entre le groupe méthyle et l'hydrogène et
c est un nombre entier de 2 à 50, et un groupe polyalkyle de structure
CH₃-(CH₂)_{d}-
dans laquelle d est un nombre entier de 25 à 50, et des triazines substituées de structure 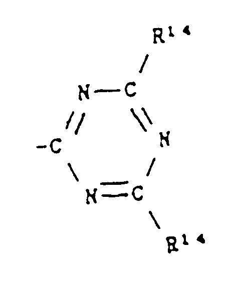 dans laquelle R¹⁴ a la définition donnée ci-dessus,
b a la valeur 0 ou 1,
R⁹ et R¹⁰ sont choisis indépendamment entre l'hydrogène, un groupe alkyle de 1 à 10 atomes de carbone, aryle de 6 à 10 atomes de carbone, et R⁹ et R¹⁰ peuvent s'associer pour former un noyau substitué ou non substitué de 5 à 12 atomes de carbone qui peut facultativement contenir une non-saturation et des hétéroatomes choisis entre l'azote, l'oxygène et le soufre,
R¹¹ et R¹² sont choisis indépendamment entre un groupe alkylène de 2 à 5 atomes de carbone, alcénylène de 2 à 3 atomes de carbone, un diradical aryle de 6 à 12 atomes de carbone et un diradical aralkyle de 7 à 15 atomes de carbone dont les diradicaux alkylène et alcénylène peuvent facultativement contenir des hétéroatomes choisis entre l'azote, l'oxygène et le soufre,
R⁷, R⁸, R⁹, R¹⁰ et R¹¹ peuvent facultativement être substitués par un ou plusieurs radicaux hydroxy, chloro, bromo, cyano, nitro, carboxy, alkoxy de 1 à 4 atomes de carbone, alkoxycarbonyle de 2 à 6 atomes de carbone et alkyle de 1 à 4 atomes de carbone, et
n a la valeur 1 ou 2.

2. Composé suivant la revendication 1, dans lequel R¹, R², R³ et R⁴ sont choisis indépendamment entre l'hydrogène, un groupe alkyle de 1 à 4 atomes de carbone substitué ou non substitué, un groupe alkoxy de 1 à 12 atomes de carbone substitué ou non substitué, un groupe aryle de 6 atomes de carbone substitué ou non substitué, un groupe aralkyle de 7 à 9 atomes de carbone substitué ou non substitue, un groupe carboxy, hydroxy ou chloro,
X est choisi entre -O-, -O-C(=O)- et une liaison simple entre le noyau et R⁵,
R⁵ est choisi entre une liaison directe et un groupe alkylène de 1 à 8 atomes de carbone substitué ou non substitué, alcénylène de 2 à 6 atomes de carbone substitué ou non substitué ou 1,2- ou 1,3- ou 1,4-phénylène substitué ou non substitué,
R⁶ est choisi entre l'hydrogène, un groupe alkyle de 1 à 4 atomes de carbone, acyle de 1 à 6 atomes de carbone et aroyle de 7 à 9 atomes de carbone,
Z est choisi entre -N(Q-R⁷)(R⁸), -N=C(R⁹)(R¹⁰), et 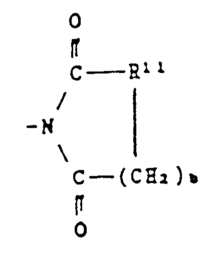 Q est choisi entre -C(=O)-, -C(=O)-O-, -C(=O)-N(R⁸)- et une liaison directe entre l'azote et R⁷,
R⁷ est choisi entre l'hydrogène, un groupe alkyle de 1 à 8 atomes de carbone, polyoxyalkylène, alcényle de 2 à 6 atomes de carbone substitué ou non substitué et aryle de 6 à 14 atomes de carbone substitué ou non substitué,
R⁸ est l'hydrogène, un groupe alkyle de 1 à 8 atomes de carbone ou aryle de 6 à 10 atomes de carbone,
b est égal à O,
R⁹ et R¹⁰ sont choisis entre l'hydrogène, un groupe alkyle de 1 à 8 atomes de carbone substitué ou non substitué, et peuvent s'associer pour former un noyau de 5 à 7 atomes de carbone substitué ou non substitué qui peut facultativement contenir une non-saturation et des hétéroatomes choisis entre l'azote, l'oxygène et le soufre, et
R¹¹ et R¹² sont choisis entre un groupe alkylène de 2 à 5 atomes de carbone, alcénylène de 2 à 3 atomes de carbone et un diradical aryle de 6 à 12 atomes de carbone.

3. Composé suivant la revendication 2, dans lequel R¹, R², R³ et R⁴ sont choisis entre l'hydrogène, un groupe alkoxy de 1 à 4 atomes de carbone substitué ou non substitué, hydroxy ou chloro,
X représente -O- ou une liaison directe entre le noyau et R⁵,
R⁵ est une liaison directe ou un groupe alkylène de 1 à 6 atomes de carbone substitué ou non substitué,
R⁶ est l'hydrogène, un groupe alkyle de 1 à 4 atomes de carbone ou acyle de 1 à 4 atomes de carbone,
Q est un groupe -C(=O)-, -C(=O)-O- et une liaison directe entre l'azote et R⁷, et
R⁷ est choisi entre l'hydrogène, un groupe alkyle de 1 à 4 atomes de carbone, polyoxyéthylène ou aryle de 6 à 14 atomes de carbone substitué ou non substitué,
R⁸ est l'hydrogène, un groupe alkyle de 1 à 4 atomes de carbone ou aryle de 6 atomes de carbone, et
R¹¹ et R¹² sont choisis indépendamment entre un groupe alkylène de 2 à 3 atomes de carbone, alcénylène de 2 atomes de carbone et arylène de 6 à 10 atomes de carbone.

4. Composé suivant la revendication 3, qui est le (4-benzoyl-3-hydroxyphénoxy)acétylhydrazide.

5. Composé suivant la revendication 3, qui est l'hydrazide d'acide 2-(2-hydroxybenzoyl)-benzoïque.

6. Composé suivant la revendication 3, qui est le 2-hydroxybenzophénone-4,4'-diyloxy-bis(acétylhydrazide).

7. Composé suivant la revendication 3, qui est le 2,2'-dihydroxybenzophénone-4,4'-diyloxy-bis(acétylhydrazide).

8. Composé suivant la revendication 3, qui est la 2,2,6,6-tétraméthyl-4-pipéridone, l'hydrazone du 4-benzoyl-3-hydroxyphénoxyacétylhydrazide ou la N-[(4-benzoyl-3-hydroxyphénoxy)acétyl]-N'-(3,5-di-tertiobutyl-4-hydroxybenzoyl)hydrazine ou le N'[(4-benzoyl-3-hydroxyphénoxy)acétyl]hydrazine-carboxylate de 2-(4-benzoyl-3-hydroxyphénoxy)éthyle.

9. Utilisation de composés suivant l'une des revendications 1 à 8 pour la stabilisation de polymères et de copolymères.

10. Composition à base d'au moins un polymère ou copolymère, caractérisée par une teneur en au moins un composé suivant les revendications 1 à 8.

## Revendications (Revendications pour l'(les) Etat(s) contractant(s) suivant(s): ES)

1. Procédé de production d'un composé de formule 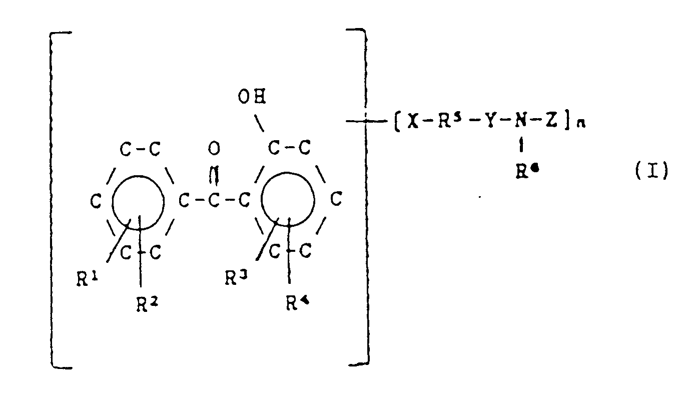 dans laquelle
R¹, R², R³ et R⁴ sont choisis, indépendamment les uns des autres, entre l'hydrogène, un groupe alkyle de 1 à 10 atomes de carbone substitué ou non substitué, un groupe acyle de 1 à 10 atomes de carbone substitué ou non substitué, un groupe alkoxy de 1 à 18 atomes de carbone substitué ou non substitué, un groupe aryle de 6 à 10 atomes de carbone substitué ou non substitué, un groupe aralkyle de 7 à 16 atomes de carbone substitué ou non substitué, un groupe alkoxycarbonyle de 2 à 19 atomes de carbone substitué ou non substitué, un groupe aryloxy de 6 à 10 atomes de carbone substitué ou non substitué, un groupe alkylmercapto de 1 à 10 atomes de carbone substitué ou non substitué, un groupe arylmercapto de 6 à 10 atomes de carbone substitué ou non substitué, un groupe alkylamino de 1 à 5 atomes de carbone substitué ou non substitué, un groupe dialkylamino de 2 à 10 atomes de carbone substitué ou non substitué, un groupe arylamino de 6 à 10 atomes de carbone substitué ou non substitué, un groupe arylalkylamino de 7 à 20 atomes de carbone substitué ou non substitué, un groupe cycloalkyle de 3 à 12 atomes de carbone substitué ou non substitué, un groupe aroyle de 7 à 11 atomes de carbone substitué ou non substitué, un groupe carboxy, hydroxy, chloro, bromo, cyano, carbamyle, sulfamyle, alkylcarbamyle ayant 1 à 10 atomes de carbone, alkylsulfamyle ayant 1 à 10 atomes de carbone, alkoxycarbonyloxy de 2 à 11 atomes de carbone, acyloxy de 1 à 10 atomes de carbone, dont les substituants sont choisis entre des substituants chloro, bromo, cyano, nitro, mercapto, alkylmercapto de 1 à 4 atomes de carbone, alkoxy de 1 à 4 atomes de carbone, hydroxy, carboxy, carbonyle, alkoxycarbonyle de 2 à 12 atomes de carbone au total et carboxylate,
X représente -O-, -N(R⁶)-, -S-, -O-C(=O)- ou une liaison directe entre le noyau aromatique et R⁵,
R⁵ est une liaison directe ou un groupe alkylène de 1 à 10 atomes de carbone, alcénylène de 2 à 10 atomes de carbone, 1,4- ou 1,3- ou 1,2-phénylène, ou cycloalcanediyle de 5 à 12 atomes de carbone, et les substituants facultatifs de ces diradicaux sont choisis entre des substituants cyano, chloro, bromo, alkyle ayant 1 à 4 atomes de carbone, acyle ayant 1 à 4 atomes de carbone, alkoxy ayant 1 à 4 atomes de carbone, carboxy, alkoxycarbonyle de 2 à 6 atomes de carbone, alkoxyalkyle de 2 à 6 atomes de carbone ou alkoxyalcénoxy de 3 à 6 atomes de carbone,
Y est choisi entre -C(=O)-, S(=O)₂,- et -OC(=O)-,
R⁶ est l'hydrogène, un groupe alkyle de 1 à 10 atomes de carbone, aryle de 6 à 10 atomes de carbone, acyle de 1 à 10 atomes de carbone ou aroyle de 7 à 11 atomes de carbone,
Z est choisi entre les groupes -N(Q-R⁷)(R⁸), -N=C(R⁹)(R¹⁰), 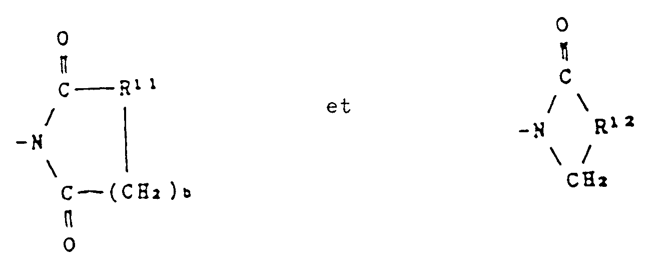 Q est choisi entre -C(=O)-, -S(=O)₂-, -C(=O)-O-, -C(=O)-N(R⁸)- et une liaison directe entre l'azote et R⁷, et
R⁷ et R⁸ sont choisis indépendamment entre l'hydrogène, un groupe alkyle de 1 à 20 atomes de carbone, alcényle de 2 à 6 atomes de carbone substitué ou non substitué, aralkyle de 7 à 12 atomes de carbone, cycloalkyle de 5 à 12 atomes de carbone, aryle de 6 à 14 atomes de carbone substitué ou non substitué et polyoxyalkylène de formule 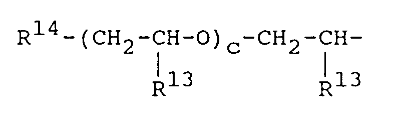 dans laquelle R¹⁴ est choisi entre un groupe alkoxy de 1 à 20 atomes de carbone, aryloxy de 6 à 10 atomes de carbone, alkoxyalkoxy de 3 à 20 atomes de carbone, alkylmercapto de 1 à 6 atomes de carbone et alcényloxy de 3 à 7 atomes de carbone,
R¹³ est choisi entre le groupe méthyle et l'hydrogène, et
c est un nombre entier de 2 à 50, et un groupe polyalkyle de structure
CH₃-(CH₂)_{d}-
dans laquelle d est un nombre entier de 25 à 50, et des triazines substituées de structure 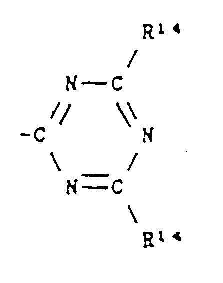 dans laquelle R¹⁴ a la définition donnée ci-dessus,
b a la valeur 0 ou 1,
R⁹ et R¹⁰ sont choisis indépendamment entre l'hydrogène, un groupe alkyle de 1 à 10 atomes de carbone, aryle de 6 à 10 atomes de carbone, et R⁹ et R¹⁰ peuvent s'associer pour former un noyau substitué ou non substitué de 5 à 12 atomes de carbone qui peut facultativement contenir une non-saturation et des hétéroatomes choisis entre l'azote, l'oxygène et le soufre,
R¹¹ et R¹² sont choisis indépendamment entre un groupe alkylène de 2 à 5 atomes de carbone, alcénylène de 2 à 3 atomes de carbone, un diradical aryle de 6 à 12 atomes de carbone et un diradical aralkyle de 7 à 15 atomes de carbone dont les diradicaux alkylène et alcénylène peuvent facultativement contenir des hétéroatomes choisis entre l'azote, l'oxygène et le soufre,
R⁷, R⁸, R⁹, R¹⁰ et R¹¹ peuvent facultativement être substitués par un ou plusieurs radicaux hydroxy, chloro, bromo, cyano, nitro, carboxy, alkoxy de 1 à 4 atomes de carbone, alkoxycarbonyle de 2 à 6 atomes de carbone et alkyle de 1 à 4 atomes de carbone, et
n a la valeur 1 ou 2,
par réaction d'un composé de formule 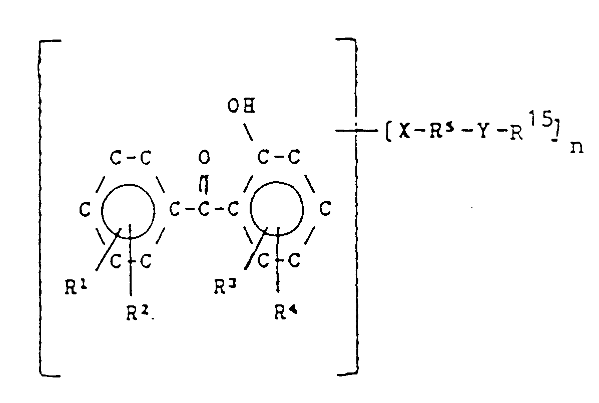 dans laquelle R¹ à R⁵, X, Y et n sont tels que précédemment définis et R¹⁵ représente H, un radical chloro ou alkyle inférieur de 1 à 6 atomes de carbone, avec l'hydrazine ou une alkyl- ou arylhydrazine pour former un composé de formule (I) que l'on peut convertir d'une manière connue en d'autres produits de formule (I).

2. Procédé suivant la revendication 1, pour la préparation d'un composé dans lequel R¹, R², R³ et R⁴ sont choisis indépendamment entre l'hydrogène, un groupe alkyle de 1 à 4 atomes de carbone substitué ou non substitué, un groupe alkoxy de 1 à 12 atomes de carbone substitué ou non substitué, un groupe aryle de 6 atomes de carbone substitué ou non substitué, un groupe aralkyle de 7 à 9 atomes de carbone substitué ou non substitué, un groupe carboxy, hydroxy ou chloro,
X est choisi entre -O-, -O-C(=O)- et une liaison directe entre le noyau et R⁵,
R⁵ est choisi entre une liaison directe et un groupe alkylène de 1 à 8 atomes de carbone substitué ou non substitué, alcénylène de 2 à 6 atomes de carbone substitué ou non substitué, ou 1,2- ou 1,3- ou 1,4-phénylène substitué ou non substitué,
R⁶ est choisi entre l'hydrogène, un groupe alkyle de 1 à 4 atomes de carbone, acyle de 1 à 6 atomes de carbone et aroyle de 7 à 9 atomes de carbone,
Z est choisi entre -N(Q-R⁷)(R⁸), -N=C(R⁹)(R¹⁰), et 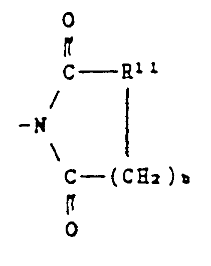 Q est choisi entre -C(=O)-, -C(=O)-O-, -C(=O)-N(R⁸)- et une liaison directe entre l'azote et R⁷,
R⁷ est choisi entre l'hydrogène, un groupe alkyle de 1 à 8 atomes de carbone, polyoxyalkylène, alcényle de 2 à 6 atomes de carbone substitué ou non substitué et aryle de 6 à 14 atomes de carbone substitué ou non substitué,
R⁸ est l'hydrogène, un groupe alkyle de 1 à 8 atomes de carbone ou aryle de 6 à 10 atomes de carbone,
b est égal à O,
R⁹ et R¹⁰ sont choisis entre l'hydrogène, un groupe alkyle de 1 à 8 atomes de carbone substitué ou non substitué, et peuvent s'associer pour former un noyau de 5 à 7 atomes de carbone substitué ou non substitué qui peut facultativement contenir une non-saturation et des hétéroatomes choisis entre l'azote, l'oxygène et le soufre, et
R¹¹ et R¹² sont choisis entre un groupe alkylène de 2 à 5 atomes de carbone, alcénylène de 2 à 3 atomes de carbone et un diradical aryle de 6 à 12 atomes de carbone.

3. Procédé suivant la revendication 2, pour la préparation d'un composé dans lequel R¹, R², R³ et R⁴ sont choisis entre l'hydrogène, un groupe alkoxy de 1 à 4 atomes de carbone substitué ou non substitué, hydroxy ou chloro,
X représente -O- ou une liaison directe entre le noyau et R⁵,
R⁵ est une liaison directe ou un groupe alkylène de 1 à 6 atomes de carbone substitué ou non substitué,
R⁶ est l'hydrogène, un groupe alkyle de 1 à 4 atomes de carbone ou acyle de 1 à 4 atomes de carbone,
Q est un groupe -C(=O)-, -C(=O)-O- et une liaison directe entre l'azote et R⁷, et
R⁷ est choisi entre l'hydrogène, un groupe alkyle de 1 à 4 atomes de carbone, polyoxyéthylène ou aryle de 6 à 14 atomes de carbone substitué ou non substitué,
R⁸ est l'hydrogène, un groupe alkyle de 1 à 4 atomes de carbone ou aryle de 6 atomes de carbone, et
R¹¹ et R¹² sont choisis indépendamment entre un groupe alkylène de 2 à 3 atomes de carbone, alcénylène de 2 atomes de carbone et arylène de 6 à 10 atomes de carbone.

4. Procédé suivant la revendication 3, pour la préparation d'un composé qui est le (4-benzoyl-3-hydroxyphénoxy)acétylhydrazide.

5. Procédé suivant la revendication 3, pour la préparation d'un composé qui est l'hydrazide d'acide 2-(2-hydroxybenzoyl)-benzoïque.

6. Procédé suivant la revendication 3, pour la préparation d'un composé qui est le 2-hydroxybenzophénone-4,4'-diyloxy-bis(acétylhydrazide).

7. Procédé suivant la revendication 3, pour la préparation d'un composé qui est le 2,2'-dihydroxybenzophénone-4,4'-diyloxy-bis(acétylhydrazide).

8. Procédé suivant la revendication 3, pour la préparation d'un composé qui est la 2,2,6,6-tétraméthyl-4-pipéridone, l'hydrazone du 4-benzoyl-3-hydroxyphénoxyacétylhydrazide ou la N-[(4-benzoyl-3-hydroxyphénoxy)acétyl]-N'-(3,5-di-tertiobutyl-4-hydroxybenzoyl)hydrazine ou le N'[(4-benzoyl-3-hydroxyphénoxy)acétyl]hydrazine-carboxylate de 2-(4-benzoyl-3-hydroxyphénoxy)éthyle.

9. Utilisation de composés préparée conformément à l'une des revendications 1 à 8 pour la stabilisation de polymères et de copolymères.

10. Composition à base d'au moins un polymère ou copolymère, caractérisée en ce qu'elle contient au moins un composé préparé conformément à l'une des revendications 1 à 8.
